# EUROPEAN PATENT SPECIFICATION

(11) **EP 2 294 202 B1**
(45) Date of publication and mention of the grant of the patent: **20.05.2015**
(21) Application number: 09793751.0
(22) Date of filing: 07.07.2009
(51) Int. Cl.: C12N 15/62, A61K 39/145, A61K 39/385, C07K 14/11, C07K 19/00, C12N 15/44, C12N 15/82, C12P 21/02

(54) **SOLUBLE RECOMBINANT INFLUENZA ANTIGENS**
LÖSLICHE REKOMBINANTE INFLUENZA-ANTIGENE
ANTIGÈNES DE LA GRIPPE RECOMBINÉS SOLUBLES

(30) Priority: 08.07.2008 US 78963 P
(43) Date of publication of application: 16.03.2011
(73) Proprietor: Medicago Inc., Québec, Québec G1S 3M7 (CA)
(72) Inventor: COUTURE, Manon, S-Augustin-de-Desmaurer Quebec G3A 2A5 (CA); LANDRY, Nathalie, St-Romuald Quebex G6W 0C1 (CA); VEZINA, Louis-Philippe, Neuville Québec G0A 2R0 (CA); DARGIS, Michèle, Quebec Quebec G2C 1M4 (CA)
(74) Representative: Kador & Partner
(86) International application number: PCT/CA2009/000941
(87) International publication number: WO 2010/003235

(56) References cited:
- WO-A1-2009/076778
- WO-A2-02/074795
- WO-A2-02/074795
- WO-A2-2008/054540
- WO-A2-2009/026397
- US-A1- 2007 286 873
- WEI CHIH-JEN ET AL: "Comparative efficacy of neutralizing antibodies elicited by recombinant hemagglutinin proteins from avian H5N1 influenza virus", JOURNAL OF VIROLOGY, THE AMERICAN SOCIETY FOR MICROBIOLOGY, US, vol. 82, no. 13, 1 July 2008 (2008-07-01), pages 6200-6208, XP009138042, ISSN: 0022-538X, DOI: 10.1128/JVI.00187-08
- VADIM METT ET AL: "A plant-produced influenza subunit vaccine protects ferrets against virus challenge", INFLUENZA AND OTHER RESPIRATORY VIRUSES, BLACKWELL PUBLISHING LTD, UK, vol. 2, no. 1, 1 January 2008 (2008-01-01) , pages 33-40, XP008130054, ISSN: 1750-2640, DOI: 10.1111/J.1750-2659.2008.00037.X [retrieved on 2008-03-28]
- SHOJI Y ET AL: "Plant-expressed HA as a seasonal influenza vaccine candidate", VACCINE, ELSEVIER LTD, GB, vol. 26, no. 23, 2 June 2008 (2008-06-02), pages 2930-2934, XP022666969, ISSN: 0264-410X, DOI: 10.1016/J.VACCINE.2008.03.045 [retrieved on 2008-04-09]
- HUANG Z ET AL: "Plant-derived measles virus hemagglutinin protein induces neutralizing antibodies in mice", VACCINE, ELSEVIER LTD, GB, vol. 19, no. 15-16, 28 February 2001 (2001-02-28), pages 2163-2171, XP008131104, ISSN: 0264-410X, DOI: 10.1016/S0264-410X(00)00390-X
- HARBURY P B ET AL: "A SWITCH BETWEEN TWO-, THREE-, AND FOUR-STRANDED COILED COILS IN GCN4 LEUCINE ZIPPER MUTANTS", SCIENCE, AMERICAN ASSOCIATION FOR THE ADVANCEMENT OF SCIENCE, WASHINGTON, DC; US, vol. 262, 26 November 1993 (1993-11-26), pages 1401-1407, XP000941971, ISSN: 0036-8075, DOI: 10.1126/SCIENCE.8248779
- WILLIAM C WELDON ET AL: "Enhanced Immunogenicity of Stabilized Trimeric Soluble Influenza Hemagglutinin", PLOS ONE, PUBLIC LIBRARY OF SCIENCE, vol. 5, no. 9, 1 September 2010 (2010-09-01), pages E12466-1, XP008136851, ISSN: 1932-6203, DOI: 10.1371/JOURNAL.PONE.0012466
- WEI, C-J. ET AL.: 'Comparative efficacy of neutralizing antibodies elicited by recombinant hemagglutinin proteins from avian H5N1 influenza virus' JOURNAL OF VIROLOGY. vol. 82, 16 April 2008, pages 6200 - 6208, XP009138042
- LUI, L. ET AL.: 'Agroinfection as a rapid method for propogating Cowpea mosaic virus-based constructs' JOURNAL OF VIROLOGICAL METHODS. vol. 105, September 2002, pages 343 - 348, XP009085158
- HUANG, Z. ET AL.: 'Plant-derived measles virus hemagglutinin protein induces neutralizing antibodies in mice' VACCINE. vol. 19, 28 February 2001, pages 2163 - 2171, XP008131104
- B. S. SHORROSH: 'Molecular Cloning of a Putative Plant Endomembrane Protein Resembling Vertebrate Protein Disulfide-Isomerase and a Phosphatidylinositol-Specific Phospholipase C' PROCEEDINGS OF THE NATIONAL ACADEMY OF SCIENCES vol. 88, no. 23, 01 December 1991, pages 10941 - 10945, XP055035646 DOI: 10.1073/pnas.88.23.10941 ISSN: 0027-8424

### SEQUENCE LISTING

<110> Medicago Inc.
   COUTURE, Manon
   VEZINA, Louis-Philippe
   DARGIS, Michele
   LANDRY, Nathalie
<120> SOLUBLE RECOMBINANT INFLUENZA ANTIGENS
<130> V81901WO
<150> 61/078,963
   <151> 2008-07-08
<160> 24
<170> PatentIn version 3.4
<210> 1
   <211> 34
   <212> PRT
   <213> Artificial
<220>
   <223> Amino acid sequence of GCN4-pII peptide
<400> 1
   Leu Lys Gln Ile Glu Asp Lys Ile Glu Glu Ile Leu Ser Lys Ile Tyr
   1 5 10 15
   His Ile Glu Asn Glu Ile Ala Arg Ile Lys Lys Leu Ile Gly Glu Ser
   20 25 30
   Ala Ala
<210> 2
   <211> 6
   <212> PRT
   <213> Artificial
<220>
   <223> Repeats of peptide PPPVHL
<400> 2
   Pro Pro Pro Val His Leu
   1 5
<210> 3
   <211> 4
   <212> PRT
   <213> Artificial
<220>
   <223> Tetrapeptide linker
<400> 3
   Gly Gly Ala Gly
   1
<210> 4
   <211> 6
   <212> PRT
   <213> Artificial
<220>
   <223> SEKDEL retention signal
<400> 4
   Ser Glu Lys Asp Glu Leu
   1 5
<210> 5
   <211> 4
   <212> PRT
   <213> Artificial
<220>
   <223> HDEL retention signal
<400> 5
   His Asp Glu Leu
   1
<210> 6
   <211> 24
   <212> PRT
   <213> Medicago sativa
<400> 6
   Met Ala Lys Asn Val Ala Ile Phe Gly Leu Leu Phe Ser Leu Leu Val
   1 5 10 15
   Leu Val Pro Ser Gln Ile Phe Ala
   20
<210> 7
   <211> 72
   <212> DNA
   <213> Medicago sativa
<400> 7
   atggcgaaaa acgttgcgat tttcggttta ttgttttctc ttcttctgtt ggttccttct 60
   cagatcttcg ct 72
<210> 8
   <211> 565
   <212> PRT
   <213> Influenza virus
<400> 8
   Met Lys Ala Lys Leu Leu Val Leu Leu Cys Thr Phe Thr Ala Thr Tyr
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asp Ser His Asn Gly Lys Leu Cys Leu Leu Lys Gly Ile
       50                  55                  60
   Ala Pro Leu Gln Leu Gly Asn Cys Ser Val Ala Gly Trp Ile Leu Gly
   65                  70                  75                  80
   Asn Pro Glu Cys Glu Leu Leu Ile Ser Lys Glu Ser Trp Ser Tyr Ile
                   85                  90                  95
   Val Glu Thr Pro Asn Pro Glu Asn Gly Thr Cys Tyr Pro Gly Tyr Phe
               100                 105                 110
   Ala Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Thr
       130                 135                 140
   Val Thr Gly Val Ser Ala Ser Cys Ser His Asn Gly Lys Ser Ser Phe
   145                 150                 155                 160
   Tyr Arg Asn Leu Leu Trp Leu Thr Gly Lys Asn Gly Leu Tyr Pro Asn
                   165                 170                 175
   Leu Ser Lys Ser Tyr Val Asn Asn Lys Glu Lys Glu Val Leu Val Leu
               180                 185                 190
   Trp Gly Val His His Pro Pro Asn Ile Gly Asn Gln Arg Ala Leu Tyr
           195                 200                 205
   His Thr Glu Asn Ala Tyr Val Ser Val Val Ser Ser His Tyr Ser Arg
       210                 215                 220
   Arg Phe Thr Pro Glu Ile Ala Lys Arg Pro Lys Val Arg Asp Gln Glu
   225                 230                 235                 240
   Gly Arg Ile Asn Tyr Tyr Trp Thr Leu Leu Glu Pro Gly Asp Thr Ile
                   245                 250                 255
   Ile Phe Glu Ala Asn Gly Asn Leu Ile Ala Pro Trp Tyr Ala Phe Ala
               260                 265                 270
   Leu Ser Arg Gly Phe Gly Ser Gly Ile Ile Thr Ser Asn Ala Pro Met
           275                 280                 285
   Asp Glu Cys Asp Ala Lys Cys Gln Thr Pro Gln Gly Ala Ile Asn Ser
       290                 295                 300
   Ser Leu Pro Phe Gln Asn Val His Pro Val Thr Ile Gly Glu Cys Pro
   305                 310                 315                 320
   Lys Tyr Val Arg Ser Ala Lys Leu Arg Met Val Thr Gly Leu Arg Asn
                   325                 330                 335
   Ile Pro Ser Ile Gln Ser Arg Gly Leu Phe Gly Ala Ile Ala Gly Phe
               340                 345                 350
   Ile Glu Gly Gly Trp Thr Gly Met Val Asp Gly Trp Tyr Gly Tyr His
           355                 360                 365
   His Gln Asn Glu Gln Gly Ser Gly Tyr Ala Ala Asp Gln Lys Ser Thr
       370                 375                 380
   Gln Asn Ala Ile Asn Gly Ile Thr Asn Lys Val Asn Ser Val Ile Glu
   385                 390                 395                 400
   Lys Met Asn Thr Gln Phe Thr Ala Val Gly Lys Glu Phe Asn Lys Leu
                   405                 410                 415
   Glu Arg Arg Met Glu Asn Leu Asn Lys Lys Val Asp Asp Gly Phe Leu
               420                 425                 430
   Asp Ile Trp Thr Tyr Asn Ala Glu Leu Leu Val Leu Leu Glu Asn Glu
           435                 440                 445
   Arg Thr Leu Asp Phe His Asp Ser Asn Val Lys Asn Leu Tyr Glu Lys
       450                 455                 460
   Val Lys Ser Gln Leu Lys Asn Asn Ala Lys Glu Ile Gly Asn Gly Cys
   465                 470                 475                 480
   Phe Glu Phe Tyr His Lys Cys Asn Asn Glu Cys Met Glu Ser Val Lys
                   485                 490                 495
   Asn Gly Thr Tyr Asp Tyr Pro Lys Tyr Ser Glu Glu Ser Lys Leu Asn
               500                 505                 510
   Arg Glu Lys Ile Asp Gly Val Lys Leu Glu Ser Met Gly Val Tyr Gln
           515                 520                 525
   Ile Leu Ala Ile Tyr Ser Thr Val Ala Ser Ser Leu Val Leu Leu Val
       530                 535                 540
   Ser Leu Gly Ala Ile Ser Phe Trp Met Cys Ser Asn Gly Ser Leu Gln
   545                 550                 555                 560
   Cys Arg Ile Cys Ile
                   565
<210> 9
   <211> 572
   <212> PRT
   <213> Artificial
<220>
   <223> Full length rHA with PDI signal peptide and transmembrane domain and cytoplasmic tail
<400> 9
   Met Ala Lys Asn Val Ala Ile Phe Gly Leu Leu Phe Ser Leu Leu Val
   1               5                   10                  15
   Leu Val Pro Ser Gln Ile Phe Ala Asp Thr Ile Cys Ile Gly Tyr His
               20                  25                  30
   Ala Asn Asn Ser Thr Asp Thr Val Asp Thr Val Leu Glu Lys Asn Val
           35                  40                  45
   Thr Val Thr His Ser Val Asn Leu Leu Glu Asp Ser His Asn Gly Lys
       50                  55                  60
   Leu Cys Leu Leu Lys Gly Ile Ala Pro Leu Gln Leu Gly Asn Cys Ser
   65                  70                  75                  80
   Val Ala Gly Trp Ile Leu Gly Asn Pro Glu Cys Glu Leu Leu Ile Ser
                   85                  90                  95
   Lys Glu Ser Trp Ser Tyr Ile Val Glu Thr Pro Asn Pro Glu Asn Gly
               100                 105                 110
   Thr Cys Tyr Pro Gly Tyr Phe Ala Asp Tyr Glu Glu Leu Arg Glu Gln
           115                 120                 125
   Leu Ser Ser Val Ser Ser Phe Glu Arg Phe Glu Ile Phe Pro Lys Glu
       130                 135                 140
   Ser Ser Trp Pro Asn His Thr Val Thr Gly Val Ser Ala Ser Cys Ser
   145                 150                 155                 160
   His Asn Gly Lys Ser Ser Phe Tyr Arg Asn Leu Leu Trp Leu Thr Gly
                   165                 170                 175
   Lys Asn Gly Leu Tyr Pro Asn Leu Ser Lys Ser Tyr Val Asn Asn Lys
               180                 185                 190
   Glu Lys Glu Val Leu Val Leu Trp Gly Val His His Pro Pro Asn Ile
           195                 200                 205
   Gly Asn Gln Arg Ala Leu Tyr His Thr Glu Asn Ala Tyr Val Ser Val
       210                 215                 220
   Val Ser Ser His Tyr Ser Arg Arg Phe Thr Pro Glu Ile Ala Lys Arg
   225                 230                 235                 240
   Pro Lys Val Arg Asp Gln Glu Gly Arg Ile Asn Tyr Tyr Trp Thr Leu
                   245                 250                 255
   Leu Glu Pro Gly Asp Thr Ile Ile Phe Glu Ala Asn Gly Asn Leu Ile
               260                 265                 270
   Ala Pro Trp Tyr Ala Phe Ala Leu Ser Arg Gly Phe Gly Ser Gly Ile
           275                 280                 285
   Ile Thr Ser Asn Ala Pro Met Asp Glu Cys Asp Ala Lys Cys Gln Thr
       290                 295                 300
   Pro Gln Gly Ala Ile Asn Ser Ser Leu Pro Phe Gln Asn Val His Pro
   305                 310                 315                 320
   Val Thr Ile Gly Glu Cys Pro Lys Tyr Val Arg Ser Ala Lys Leu Arg
                   325                 330                 335
   Met Val Thr Gly Leu Arg Asn Ile Pro Ser Ile Gln Ser Arg Gly Leu
               340                 345                 350
   Phe Gly Ala Ile Ala Gly Phe Ile Glu Gly Gly Trp Thr Gly Met Val
           355                 360                 365
   Asp Gly Trp Tyr Gly Tyr His His Gln Asn Glu Gln Gly Ser Gly Tyr
       370                 375                 380
   Ala Ala Asp Gln Lys Ser Thr Gln Asn Ala Ile Asn Gly Ile Thr Asn
   385                 390                 395                 400
   Lys Val Asn Ser Val Ile Glu Lys Met Asn Thr Gln Phe Thr Ala Val
                   405                 410                 415
   Gly Lys Glu Phe Asn Lys Leu Glu Arg Arg Met Glu Asn Leu Asn Lys
               420                 425                 430
   Lys Val Asp Asp Gly Phe Leu Asp Ile Trp Thr Tyr Asn Ala Glu Leu
           435                 440                 445
   Leu Val Leu Leu Glu Asn Glu Arg Thr Leu Asp Phe His Asp Ser Asn
       450                 455                 460
   Val Lys Asn Leu Tyr Glu Lys Val Lys Ser Gln Leu Lys Asn Asn Ala
   465                 470                 475                 480
   Lys Glu Ile Gly Asn Gly Cys Phe Glu Phe Tyr His Lys Cys Asn Asn
                   485                 490                 495
   Glu Cys Met Glu Ser Val Lys Asn Gly Thr Tyr Asp Tyr Pro Lys Tyr
               500                 505                 510
   Ser Glu Glu Ser Lys Leu Asn Arg Glu Lys Ile Asp Gly Val Lys Leu
           515                 520                 525
   Glu Ser Met Gly Val Tyr Gln Ile Leu Ala Ile Tyr Ser Thr Val Ala
       530                 535                 540
   Ser Ser Leu Val Leu Leu Val Ser Leu Gly Ala Ile Ser Phe Trp Met
   545                 550                 555                 560
   Cys Ser Asn Gly Ser Leu Gln Cys Arg Ile Cys Ile
                   565                 570
<210> 10
   <211> 540
   <212> PRT
   <213> Artificial
<220>
   <223> ER-retained rHA using SEKDEL retention signal
<400> 10
   Met Ala Lys Asn Val Ala Ile Phe Gly Leu Leu Phe Ser Leu Leu Val
   1               5                   10                  15
   Leu Val Pro Ser Gln Ile Phe Ala Asp Thr Ile Cys Ile Gly Tyr His
               20                  25                  30
   Ala Asn Asn Ser Thr Asp Thr Val Asp Thr Val Leu Glu Lys Asn Val
           35                  40                  45
   Thr Val Thr His Ser Val Asn Leu Leu Glu Asp Ser His Asn Gly Lys
       50                  55                  60
   Leu Cys Leu Leu Lys Gly Ile Ala Pro Leu Gln Leu Gly Asn Cys Ser
   65                  70                  75                  80
   Val Ala Gly Trp Ile Leu Gly Asn Pro Glu Cys Glu Leu Leu Ile Ser
                   85                  90                  95
   Lys Glu Ser Trp Ser Tyr Ile Val Glu Thr Pro Asn Pro Glu Asn Gly
               100                 105                 110
   Thr Cys Tyr Pro Gly Tyr Phe Ala Asp Tyr Glu Glu Leu Arg Glu Gln
           115                 120                 125
   Leu Ser Ser Val Ser Ser Phe Glu Arg Phe Glu Ile Phe Pro Lys Glu
       130                 135                 140
   Ser Ser Trp Pro Asn His Thr Val Thr Gly Val Ser Ala Ser Cys Ser
   145                 150                 155                 160
   His Asn Gly Lys Ser Ser Phe Tyr Arg Asn Leu Leu Trp Leu Thr Gly
                   165                 170                 175
   Lys Asn Gly Leu Tyr Pro Asn Leu Ser Lys Ser Tyr Val Asn Asn Lys
               180                 185                 190
   Glu Lys Glu Val Leu Val Leu Trp Gly Val His His Pro Pro Asn Ile
           195                 200                 205
   Gly Asn Gln Arg Ala Leu Tyr His Thr Glu Asn Ala Tyr Val Ser Val
       210                 215                 220
   Val Ser Ser His Tyr Ser Arg Arg Phe Thr Pro Glu Ile Ala Lys Arg
   225                 230                 235                 240
   Pro Lys Val Arg Asp Gln Glu Gly Arg Ile Asn Tyr Tyr Trp Thr Leu
                   245                 250                 255
   Leu Glu Pro Gly Asp Thr Ile Ile Phe Glu Ala Asn Gly Asn Leu Ile
               260                 265                 270
   Ala Pro Trp Tyr Ala Phe Ala Leu Ser Arg Gly Phe Gly Ser Gly Ile
           275                 280                 285
   Ile Thr Ser Asn Ala Pro Met Asp Glu Cys Asp Ala Lys Cys Gln Thr
       290                 295                 300
   Pro Gln Gly Ala Ile Asn Ser Ser Leu Pro Phe Gln Asn Val His Pro
   305                 310                 315                 320
   Val Thr Ile Gly Glu Cys Pro Lys Tyr Val Arg Ser Ala Lys Leu Arg
   325 330 335
   Met Val Thr Gly Leu Arg Asn Ile Pro Ser Ile Gln Ser Arg Gly Leu
   340 345 350
   Phe Gly Ala Ile Ala Gly Phe Ile Glu Gly Gly Trp Thr Gly Met Val
   355 360 365
   Asp Gly Trp Tyr Gly Tyr His His Gln Asn Glu Gln Gly Ser Gly Tyr
   370 375 380
   Ala Ala Asp Gln Lys Ser Thr Gln Asn Ala Ile Asn Gly Ile Thr Asn
   385 390 395 400
   Lys Val Asn Ser Val Ile Glu Lys Met Asn Thr Gln Phe Thr Ala Val
   405 410 415
   Gly Lys Glu Phe Asn Lys Leu Glu Arg Arg Met Glu Asn Leu Asn Lys
   420 425 430
   Lys Val Asp Asp Gly Phe Leu Asp Ile Trp Thr Tyr Asn Ala Glu Leu
   435 440 445
   Leu Val Leu Leu Glu Asn Glu Arg Thr Leu Asp Phe His Asp Ser Asn
   450 455 460
   Val Lys Asn Leu Tyr Glu Lys Val Lys Ser Gln Leu Lys Asn Asn Ala
   465 470 475 480
   Lys Glu Ile Gly Asn Gly Cys Phe Glu Phe Tyr His Lys Cys Asn Asn
   485 490 495
   Glu Cys Met Glu Ser Val Lys Asn Gly Thr Tyr Asp Tyr Pro Lys Tyr
   500 505 510
   Ser Glu Glu Ser Lys Leu Asn Arg Glu Lys Ile Asp Gly Val Lys Leu
   515 520 525
   Glu Ser Met Gly Val Tyr Ser Glu Lys Asp Glu Leu
   530 535 540
<210> 11
   <211> 538
   <212> PRT
   <213> Artificial
<220>
   <223> ER-retained rHA using HDEL retention signal
<400> 11
   Met Ala Lys Asn Val Ala Ile Phe Gly Leu Leu Phe Ser Leu Leu Val
   1               5                   10                  15
   Leu Val Pro Ser Gln Ile Phe Ala Asp Thr Ile Cys Ile Gly Tyr His
               20                  25                  30
   Ala Asn Asn Ser Thr Asp Thr Val Asp Thr Val Leu Glu Lys Asn Val
           35                  40                  45
   Thr Val Thr His Ser Val Asn Leu Leu Glu Asp Ser His Asn Gly Lys
       50                  55                  60
   Leu Cys Leu Leu Lys Gly Ile Ala Pro Leu Gln Leu Gly Asn Cys Ser
   65                  70                  75                  80
   Val Ala Gly Trp Ile Leu Gly Asn Pro Glu Cys Glu Leu Leu Ile Ser
                   85                  90                  95
   Lys Glu Ser Trp Ser Tyr Ile Val Glu Thr Pro Asn Pro Glu Asn Gly
               100                 105                 110
   Thr Cys Tyr Pro Gly Tyr Phe Ala Asp Tyr Glu Glu Leu Arg Glu Gln
           115                 120                 125
   Leu Ser Ser Val Ser Ser Phe Glu Arg Phe Glu Ile Phe Pro Lys Glu
       130                 135                 140
   Ser Ser Trp Pro Asn His Thr Val Thr Gly Val Ser Ala Ser Cys Ser
   145                 150                 155                 160
   His Asn Gly Lys Ser Ser Phe Tyr Arg Asn Leu Leu Trp Leu Thr Gly
                   165                 170                 175
   Lys Asn Gly Leu Tyr Pro Asn Leu Ser Lys Ser Tyr Val Asn Asn Lys
               180                 185                 190
   Glu Lys Glu Val Leu Val Leu Trp Gly Val His His Pro Pro Asn Ile
           195                 200                 205
   Gly Asn Gln Arg Ala Leu Tyr His Thr Glu Asn Ala Tyr Val Ser Val
       210                 215                 220
   Val Ser Ser His Tyr Ser Arg Arg Phe Thr Pro Glu Ile Ala Lys Arg
   225                 230                 235                 240
   Pro Lys Val Arg Asp Gln Glu Gly Arg Ile Asn Tyr Tyr Trp Thr Leu
                   245                 250                 255
   Leu Glu Pro Gly Asp Thr Ile Ile Phe Glu Ala Asn Gly Asn Leu Ile
               260                 265                 270
   Ala Pro Trp Tyr Ala Phe Ala Leu Ser Arg Gly Phe Gly Ser Gly Ile
           275                 280                 285
   Ile Thr Ser Asn Ala Pro Met Asp Glu Cys Asp Ala Lys Cys Gln Thr
       290                 295                 300
   Pro Gln Gly Ala Ile Asn Ser Ser Leu Pro Phe Gln Asn Val His Pro
   305                 310                 315                 320
   Val Thr Ile Gly Glu Cys Pro Lys Tyr Val Arg Ser Ala Lys Leu Arg
                   325                 330                 335
   Met Val Thr Gly Leu Arg Asn Ile Pro Ser Ile Gln Ser Arg Gly Leu
               340                 345                 350
   Phe Gly Ala Ile Ala Gly Phe Ile Glu Gly Gly Trp Thr Gly Met Val
           355                 360                 365
   Asp Gly Trp Tyr Gly Tyr His His Gln Asn Glu Gln Gly Ser Gly Tyr
       370                 375                 380
   Ala Ala Asp Gln Lys Ser Thr Gln Asn Ala Ile Asn Gly Ile Thr Asn
   385                 390                 395                 400
   Lys Val Asn Ser Val Ile Glu Lys Met Asn Thr Gln Phe Thr Ala Val
                   405                 410                 415
   Gly Lys Glu Phe Asn Lys Leu Glu Arg Arg Met Glu Asn Leu Asn Lys
               420                 425                 430
   Lys Val Asp Asp Gly Phe Leu Asp Ile Trp Thr Tyr Asn Ala Glu Leu
           435                 440                 445
   Leu Val Leu Leu Glu Asn Glu Arg Thr Leu Asp Phe His Asp Ser Asn
       450                 455                 460
   Val Lys Asn Leu Tyr Glu Lys Val Lys Ser Gln Leu Lys Asn Asn Ala
   465                 470                 475                 480
   Lys Glu Ile Gly Asn Gly Cys Phe Glu Phe Tyr His Lys Cys Asn Asn
                   485                 490                 495
   Glu Cys Met Glu Ser Val Lys Asn Gly Thr Tyr Asp Tyr Pro Lys Tyr
               500                 505                 510
   Ser Glu Glu Ser Lys Leu Asn Arg Glu Lys Ile Asp Gly Val Lys Leu
           515                 520                 525
   Glu Ser Met Gly Val Tyr His Asp Glu Leu
       530                 535
<210> 12
   <211> 534
   <212> PRT
   <213> Artificial
<220>
   <223> Soluble rHA without transmembrane domain
<400> 12
   Met Ala Lys Asn Val Ala Ile Phe Gly Leu Leu Phe Ser Leu Leu Val
   1               5                   10                  15
   Leu Val Pro Ser Gln Ile Phe Ala Asp Thr Ile Cys Ile Gly Tyr His
               20                  25                  30
   Ala Asn Asn Ser Thr Asp Thr Val Asp Thr Val Leu Glu Lys Asn Val
           35                  40                  45
   Thr Val Thr His Ser Val Asn Leu Leu Glu Asp Ser His Asn Gly Lys
       50                  55                  60
   Leu Cys Leu Leu Lys Gly Ile Ala Pro Leu Gln Leu Gly Asn Cys Ser
   65                  70                  75                  80
   Val Ala Gly Trp Ile Leu Gly Asn Pro Glu Cys Glu Leu Leu Ile Ser
                   85                  90                  95
   Lys Glu Ser Trp Ser Tyr Ile Val Glu Thr Pro Asn Pro Glu Asn Gly
               100                 105                 110
   Thr Cys Tyr Pro Gly Tyr Phe Ala Asp Tyr Glu Glu Leu Arg Glu Gln
           115                 120                 125
   Leu Ser Ser Val Ser Ser Phe Glu Arg Phe Glu Ile Phe Pro Lys Glu
       130                 135                 140
   Ser Ser Trp Pro Asn His Thr Val Thr Gly Val Ser Ala Ser Cys Ser
   145                 150                 155                 160
   His Asn Gly Lys Ser Ser Phe Tyr Arg Asn Leu Leu Trp Leu Thr Gly
                   165                 170                 175
   Lys Asn Gly Leu Tyr Pro Asn Leu Ser Lys Ser Tyr Val Asn Asn Lys
               180                 185                 190
   Glu Lys Glu Val Leu Val Leu Trp Gly Val His His Pro Pro Asn Ile
           195                 200                 205
   Gly Asn Gln Arg Ala Leu Tyr His Thr Glu Asn Ala Tyr Val Ser Val
       210                 215                 220
   Val Ser Ser His Tyr Ser Arg Arg Phe Thr Pro Glu Ile Ala Lys Arg
   225                 230                 235                 240
   Pro Lys Val Arg Asp Gln Glu Gly Arg Ile Asn Tyr Tyr Trp Thr Leu
                   245                 250                 255
   Leu Glu Pro Gly Asp Thr Ile Ile Phe Glu Ala Asn Gly Asn Leu Ile
               260                 265                 270
   Ala Pro Trp Tyr Ala Phe Ala Leu Ser Arg Gly Phe Gly Ser Gly Ile
           275                 280                 285
   Ile Thr Ser Asn Ala Pro Met Asp Glu Cys Asp Ala Lys Cys Gln Thr
       290                 295                 300
   Pro Gln Gly Ala Ile Asn Ser Ser Leu Pro Phe Gln Asn Val His Pro
   305                 310                 315                 320
   Val Thr Ile Gly Glu Cys Pro Lys Tyr Val Arg Ser Ala Lys Leu Arg
                   325                 330                 335
   Met Val Thr Gly Leu Arg Asn Ile Pro Ser Ile Gln Ser Arg Gly Leu
               340                 345                 350
   Phe Gly Ala Ile Ala Gly Phe Ile Glu Gly Gly Trp Thr Gly Met Val
           355                 360                 365
   Asp Gly Trp Tyr Gly Tyr His His Gln Asn Glu Gln Gly Ser Gly Tyr
       370                 375                 380
   Ala Ala Asp Gln Lys Ser Thr Gln Asn Ala Ile Asn Gly Ile Thr Asn
   385                 390                 395                 400
   Lys Val Asn Ser Val Ile Glu Lys Met Asn Thr Gln Phe Thr Ala Val
                   405                 410                 415
   Gly Lys Glu Phe Asn Lys Leu Glu Arg Arg Met Glu Asn Leu Asn Lys
               420                 425                 430
   Lys Val Asp Asp Gly Phe Leu Asp Ile Trp Thr Tyr Asn Ala Glu Leu
           435                 440                 445
   Leu Val Leu Leu Glu Asn Glu Arg Thr Leu Asp Phe His Asp Ser Asn
       450                 455                 460
   Val Lys Asn Leu Tyr Glu Lys Val Lys Ser Gln Leu Lys Asn Asn Ala
   465                 470                 475                 480
   Lys Glu Ile Gly Asn Gly Cys Phe Glu Phe Tyr His Lys Cys Asn Asn
                   485                 490                 495
   Glu Cys Met Glu Ser Val Lys Asn Gly Thr Tyr Asp Tyr Pro Lys Tyr
               500                 505                 510
   Ser Glu Glu Ser Lys Leu Asn Arg Glu Lys Ile Asp Gly Val Lys Leu
           515                 520                 525
   Glu Ser Met Gly Val Tyr
       530
<210> 13
   <211> 568
   <212> PRT
   <213> Artificial
<220>
   <223> Soluble trimeric rHA using GCN4-pII trimeric peptide
<400> 13
   Met Ala Lys Asn Val Ala Ile Phe Gly Leu Leu Phe Ser Leu Leu Val
   1               5                   10                  15
   Leu Val Pro Ser Gln Ile Phe Ala Asp Thr Ile Cys Ile Gly Tyr His
               20                  25                  30
   Ala Asn Asn Ser Thr Asp Thr Val Asp Thr Val Leu Glu Lys Asn Val
           35                  40                  45
   Thr Val Thr His Ser Val Asn Leu Leu Glu Asp Ser His Asn Gly Lys
       50                  55                  60
   Leu Cys Leu Leu Lys Gly Ile Ala Pro Leu Gln Leu Gly Asn Cys Ser
   65                  70                  75                  80
   Val Ala Gly Trp Ile Leu Gly Asn Pro Glu Cys Glu Leu Leu Ile Ser
                   85                  90                  95
   Lys Glu Ser Trp Ser Tyr Ile Val Glu Thr Pro Asn Pro Glu Asn Gly
               100                 105                 110
   Thr Cys Tyr Pro Gly Tyr Phe Ala Asp Tyr Glu Glu Leu Arg Glu Gln
           115                 120                 125
   Leu Ser Ser Val Ser Ser Phe Glu Arg Phe Glu Ile Phe Pro Lys Glu
       130                 135                 140
   Ser Ser Trp Pro Asn His Thr Val Thr Gly Val Ser Ala Ser Cys Ser
   145                 150                 155                 160
   His Asn Gly Lys Ser Ser Phe Tyr Arg Asn Leu Leu Trp Leu Thr Gly
                   165                 170                 175
   Lys Asn Gly Leu Tyr Pro Asn Leu Ser Lys Ser Tyr Val Asn Asn Lys
               180                 185                 190
   Glu Lys Glu Val Leu Val Leu Trp Gly Val His His Pro Pro Asn Ile
           195                 200                 205
   Gly Asn Gln Arg Ala Leu Tyr His Thr Glu Asn Ala Tyr Val Ser Val
       210                 215                 220
   Val Ser Ser His Tyr Ser Arg Arg Phe Thr Pro Glu Ile Ala Lys Arg
   225                 230                 235                 240
   Pro Lys Val Arg Asp Gln Glu Gly Arg Ile Asn Tyr Tyr Trp Thr Leu
                   245                 250                 255
   Leu Glu Pro Gly Asp Thr Ile Ile Phe Glu Ala Asn Gly Asn Leu Ile
               260                 265                 270
   Ala Pro Trp Tyr Ala Phe Ala Leu Ser Arg Gly Phe Gly Ser Gly Ile
           275                 280                 285
   Ile Thr Ser Asn Ala Pro Met Asp Glu Cys Asp Ala Lys Cys Gln Thr
       290                 295                 300
   Pro Gln Gly Ala Ile Asn Ser Ser Leu Pro Phe Gln Asn Val His Pro
   305                 310                 315                 320
   Val Thr Ile Gly Glu Cys Pro Lys Tyr Val Arg Ser Ala Lys Leu Arg
                   325                 330                 335
   Met Val Thr Gly Leu Arg Asn Ile Pro Ser Ile Gln Ser Arg Gly Leu
               340                 345                 350
   Phe Gly Ala Ile Ala Gly Phe Ile Glu Gly Gly Trp Thr Gly Met Val
           355                 360                 365
   Asp Gly Trp Tyr Gly Tyr His His Gln Asn Glu Gln Gly Ser Gly Tyr
       370                 375                 380
   Ala Ala Asp Gln Lys Ser Thr Gln Asn Ala Ile Asn Gly Ile Thr Asn
   385                 390                 395                 400
   Lys Val Asn Ser Val Ile Glu Lys Met Asn Thr Gln Phe Thr Ala Val
                   405                 410                 415
   Gly Lys Glu Phe Asn Lys Leu Glu Arg Arg Met Glu Asn Leu Asn Lys
               420                 425                 430
   Lys Val Asp Asp Gly Phe Leu Asp Ile Trp Thr Tyr Asn Ala Glu Leu
           435                 440                 445
   Leu Val Leu Leu Glu Asn Glu Arg Thr Leu Asp Phe His Asp Ser Asn
       450                 455                 460
   Val Lys Asn Leu Tyr Glu Lys Val Lys Ser Gln Leu Lys Asn Asn Ala
   465 470 475 480
   Lys Glu Ile Gly Asn Gly Cys Phe Glu Phe Tyr His Lys Cys Asn Asn
   485 490 495
   Glu Cys Met Glu Ser Val Lys Asn Gly Thr Tyr Asp Tyr Pro Lys Tyr
   500 505 510
   Ser Glu Glu Ser Lys Leu Asn Arg Glu Lys Ile Asp Gly Val Lys Leu
   515 520 525
   Glu Ser Met Gly Val Tyr Leu Lys Gln Ile Glu Asp Lys Ile Glu Glu
   530 535 540
   Ile Leu Ser Lys Ile Tyr His Ile Glu Asn Glu Ile Ala Arg Ile Lys
   545 550 555 560
   Lys Leu Ile Gly Glu Ser Ala Ala
   565
<210> 14
   <211> 574
   <212> PRT
   <213> Artificial
<220>
   <223> Soluble trimeric rHA using GCN4-pII trimeric
   peptide and retained in the ER
<400> 14
   Met Ala Lys Asn Val Ala Ile Phe Gly Leu Leu Phe Ser Leu Leu Val
   1 5 10 15
   Leu Val Pro Ser Gln Ile Phe Ala Asp Thr Ile Cys Ile Gly Tyr His
   20 25 30
   Ala Asn Asn Ser Thr Asp Thr Val Asp Thr Val Leu Glu Lys Asn Val
   35 40 45
   Thr Val Thr His Ser Val Asn Leu Leu Glu Asp Ser His Asn Gly Lys
   50 55 60
   Leu Cys Leu Leu Lys Gly Ile Ala Pro Leu Gln Leu Gly Asn Cys Ser
   65 70 75 80
   Val Ala Gly Trp Ile Leu Gly Asn Pro Glu Cys Glu Leu Leu Ile Ser
   85 90 95
   Lys Glu Ser Trp Ser Tyr Ile Val Glu Thr Pro Asn Pro Glu Asn Gly
   100 105 110
   Thr Cys Tyr Pro Gly Tyr Phe Ala Asp Tyr Glu Glu Leu Arg Glu Gln
   115 120 125
   Leu Ser Ser Val Ser Ser Phe Glu Arg Phe Glu Ile Phe Pro Lys Glu
   130 135 140
   Ser Ser Trp Pro Asn His Thr Val Thr Gly Val Ser Ala Ser Cys Ser
   145 150 155 160
   His Asn Gly Lys Ser Ser Phe Tyr Arg Asn Leu Leu Trp Leu Thr Gly
   165 170 175
   Lys Asn Gly Leu Tyr Pro Asn Leu Ser Lys Ser Tyr Val Asn Asn Lys
   180 185 190
   Glu Lys Glu Val Leu Val Leu Trp Gly Val His His Pro Pro Asn Ile
   195 200 205
   Gly Asn Gln Arg Ala Leu Tyr His Thr Glu Asn Ala Tyr Val Ser Val
   210 215 220
   Val Ser Ser His Tyr Ser Arg Arg Phe Thr Pro Glu Ile Ala Lys Arg
   225 230 235 240
   Pro Lys Val Arg Asp Gln Glu Gly Arg Ile Asn Tyr Tyr Trp Thr Leu
   245 250 255
   Leu Glu Pro Gly Asp Thr Ile Ile Phe Glu Ala Asn Gly Asn Leu Ile
   260 265 270
   Ala Pro Trp Tyr Ala Phe Ala Leu Ser Arg Gly Phe Gly Ser Gly Ile
   275 280 285
   Ile Thr Ser Asn Ala Pro Met Asp Glu Cys Asp Ala Lys Cys Gln Thr
   290 295 300
   Pro Gln Gly Ala Ile Asn Ser Ser Leu Pro Phe Gln Asn Val His Pro
   305 310 315 320
   Val Thr Ile Gly Glu Cys Pro Lys Tyr Val Arg Ser Ala Lys Leu Arg
   325 330 335
   Met Val Thr Gly Leu Arg Asn Ile Pro Ser Ile Gln Ser Arg Gly Leu
   340 345 350
   Phe Gly Ala Ile Ala Gly Phe Ile Glu Gly Gly Trp Thr Gly Met Val
   355 360 365
   Asp Gly Trp Tyr Gly Tyr His His Gln Asn Glu Gln Gly Ser Gly Tyr
   370 375 380
   Ala Ala Asp Gln Lys Ser Thr Gln Asn Ala Ile Asn Gly Ile Thr Asn
   385 390 395 400
   Lys Val Asn Ser Val Ile Glu Lys Met Asn Thr Gln Phe Thr Ala Val
   405 410 415
   Gly Lys Glu Phe Asn Lys Leu Glu Arg Arg Met Glu Asn Leu Asn Lys
   420 425 430
   Lys Val Asp Asp Gly Phe Leu Asp Ile Trp Thr Tyr Asn Ala Glu Leu
   435 440 445
   Leu Val Leu Leu Glu Asn Glu Arg Thr Leu Asp Phe His Asp Ser Asn
   450 455 460
   Val Lys Asn Leu Tyr Glu Lys Val Lys Ser Gln Leu Lys Asn Asn Ala
   465                 470                 475                 480
   Lys Glu Ile Gly Asn Gly Cys Phe Glu Phe Tyr His Lys Cys Asn Asn
                   485                 490                 495
   Glu Cys Met Glu Ser Val Lys Asn Gly Thr Tyr Asp Tyr Pro Lys Tyr
               500                 505                 510
   Ser Glu Glu Ser Lys Leu Asn Arg Glu Lys Ile Asp Gly Val Lys Leu
           515                 520                 525
   Glu Ser Met Gly Val Tyr Leu Lys Gln Ile Glu Asp Lys Ile Glu Glu
       530                 535                 540
   Ile Leu Ser Lys Ile Tyr His Ile Glu Asn Glu Ile Ala Arg Ile Lys
   545                 550                 555                 560
   Lys Leu Ile Gly Glu Ser Ala Ala Ser Glu Asp Lys Glu Leu
                   565                 570
<210> 15
   <211> 593
   <212> PRT
   <213> Artificial
<220>
   <223> Soluble trimeric rHA using PRD trimeric peptide
<400> 15
   Met Ala Lys Asn Val Ala Ile Phe Gly Leu Leu Phe Ser Leu Leu Val
   1               5                   10                  15
   Leu Val Pro Ser Gln Ile Phe Ala Asp Thr Ile Cys Ile Gly Tyr His
               20                  25                  30
   Ala Asn Asn Ser Thr Asp Thr Val Asp Thr Val Leu Glu Lys Asn Val
           35                  40                  45
   Thr Val Thr His Ser Val Asn Leu Leu Glu Asp Ser His Asn Gly Lys
       50                  55                  60
   Leu Cys Leu Leu Lys Gly Ile Ala Pro Leu Gln Leu Gly Asn Cys Ser
   65                  70                  75                  80
   Val Ala Gly Trp Ile Leu Gly Asn Pro Glu Cys Glu Leu Leu Ile Ser
                   85                  90                  95
   Lys Glu Ser Trp Ser Tyr Ile Val Glu Thr Pro Asn Pro Glu Asn Gly
               100                 105                 110
   Thr Cys Tyr Pro Gly Tyr Phe Ala Asp Tyr Glu Glu Leu Arg Glu Gln
           115                 120                 125
   Leu Ser Ser Val Ser Ser Phe Glu Arg Phe Glu Ile Phe Pro Lys Glu
       130                 135                 140
   Ser Ser Trp Pro Asn His Thr Val Thr Gly Val Ser Ala Ser Cys Ser
   145                 150                 155                 160
   His Asn Gly Lys Ser Ser Phe Tyr Arg Asn Leu Leu Trp Leu Thr Gly
   165 170 175
   Lys Asn Gly Leu Tyr Pro Asn Leu Ser Lys Ser Tyr Val Asn Asn Lys
   180 185 190
   Glu Lys Glu Val Leu Val Leu Trp Gly Val His His Pro Pro Asn Ile
   195 200 205
   Gly Asn Gln Arg Ala Leu Tyr His Thr Glu Asn Ala Tyr Val Ser Val
   210 215 220
   Val Ser Ser His Tyr Ser Arg Arg Phe Thr Pro Glu Ile Ala Lys Arg
   225 230 235 240
   Pro Lys Val Arg Asp Gln Glu Gly Arg Ile Asn Tyr Tyr Trp Thr Leu
   245 250 255
   Leu Glu Pro Gly Asp Thr Ile Ile Phe Glu Ala Asn Gly Asn Leu Ile
   260 265 270
   Ala Pro Trp Tyr Ala Phe Ala Leu Ser Arg Gly Phe Gly Ser Gly Ile
   275 280 285
   Ile Thr Ser Asn Ala Pro Met Asp Glu Cys Asp Ala Lys Cys Gln Thr
   290 295 300
   Pro Gln Gly Ala Ile Asn Ser Ser Leu Pro Phe Gln Asn Val His Pro
   305 310 315 320
   Val Thr Ile Gly Glu Cys Pro Lys Tyr Val Arg Ser Ala Lys Leu Arg
   325 330 335
   Met Val Thr Gly Leu Arg Asn Ile Pro Ser Ile Gln Ser Arg Gly Leu
   340 345 350
   Phe Gly Ala Ile Ala Gly Phe Ile Glu Gly Gly Trp Thr Gly Met Val
   355 360 365
   Asp Gly Trp Tyr Gly Tyr His His Gln Asn Glu Gln Gly Ser Gly Tyr
   370 375 380
   Ala Ala Asp Gln Lys Ser Thr Gln Asn Ala Ile Asn Gly Ile Thr Asn
   385 390 395 400
   Lys Val Asn Ser Val Ile Glu Lys Met Asn Thr Gln Phe Thr Ala Val
   405 410 415
   Gly Lys Glu Phe Asn Lys Leu Glu Arg Arg Met Glu Asn Leu Asn Lys
   420 425 430
   Lys Val Asp Asp Gly Phe Leu Asp Ile Trp Thr Tyr Asn Ala Glu Leu
   435 440 445
   Leu Val Leu Leu Glu Asn Glu Arg Thr Leu Asp Phe His Asp Ser Asn
   450 455 460
   Val Lys Asn Leu Tyr Glu Lys Val Lys Ser Gln Leu Lys Asn Asn Ala
   465 470 475 480
   Lys Glu Ile Gly Asn Gly Cys Phe Glu Phe Tyr His Lys Cys Asn Asn
                   485                 490                 495
   Glu Cys Met Glu Ser Val Lys Asn Gly Thr Tyr Asp Tyr Pro Lys Tyr
               500                 505                 510
   Ser Glu Glu Ser Lys Leu Asn Arg Glu Lys Ile Asp Gly Val Lys Leu
           515                 520                 525
   Glu Ser Met Gly Val Tyr Gly Gly Ala Gly Pro Pro Pro Pro Val His
       530                 535                 540
   Leu Pro Pro Pro Val His Leu Pro Pro Pro Val His Leu Pro Pro Pro
   545                 550                 555                 560
   Val His Leu Pro Pro Pro Val His Leu Pro Pro Pro Val His Leu Pro
                   565                 570                 575
   Pro Pro Val His Leu Pro Pro Pro Val His Leu Pro Pro Pro Ser Ala
               580                 585                 590
   Ala
<210> 16
   <211> 599
   <212> PRT
   <213> Artificial
<220>
   <223> Soluble trimeric rHA using RDP trimeric peptide
   and retained in ER
<400> 16
   Met Ala Lys Asn Val Ala Ile Phe Gly Leu Leu Phe Ser Leu Leu Val
   1               5                   10                  15
   Leu Val Pro Ser Gln Ile Phe Ala Asp Thr Ile Cys Ile Gly Tyr His
               20                  25                  30
   Ala Asn Asn Ser Thr Asp Thr Val Asp Thr Val Leu Glu Lys Asn Val
           35                  40                  45
   Thr Val Thr His Ser Val Asn Leu Leu Glu Asp Ser His Asn Gly Lys
       50                  55                  60
   Leu Cys Leu Leu Lys Gly Ile Ala Pro Leu Gln Leu Gly Asn Cys Ser
   65                  70                  75                  80
   Val Ala Gly Trp Ile Leu Gly Asn Pro Glu Cys Glu Leu Leu Ile Ser
                   85                  90                  95
   Lys Glu Ser Trp Ser Tyr Ile Val Glu Thr Pro Asn Pro Glu Asn Gly
               100                 105                 110
   Thr Cys Tyr Pro Gly Tyr Phe Ala Asp Tyr Glu Glu Leu Arg Glu Gln
           115                 120                 125
   Leu Ser Ser Val Ser Ser Phe Glu Arg Phe Glu Ile Phe Pro Lys Glu
       130                 135                 140
   Ser Ser Trp Pro Asn His Thr Val Thr Gly Val Ser Ala Ser Cys Ser
   145                 150                 155                 160
   His Asn Gly Lys Ser Ser Phe Tyr Arg Asn Leu Leu Trp Leu Thr Gly
                   165                 170                 175
   Lys Asn Gly Leu Tyr Pro Asn Leu Ser Lys Ser Tyr Val Asn Asn Lys
               180                 185                 190
   Glu Lys Glu Val Leu Val Leu Trp Gly Val His His Pro Pro Asn Ile
           195                 200                 205
   Gly Asn Gln Arg Ala Leu Tyr His Thr Glu Asn Ala Tyr Val Ser Val
       210                 215                 220
   Val Ser Ser His Tyr Ser Arg Arg Phe Thr Pro Glu Ile Ala Lys Arg
   225                 230                 235                 240
   Pro Lys Val Arg Asp Gln Glu Gly Arg Ile Asn Tyr Tyr Trp Thr Leu
                   245                 250                 255
   Leu Glu Pro Gly Asp Thr Ile Ile Phe Glu Ala Asn Gly Asn Leu Ile
               260                 265                 270
   Ala Pro Trp Tyr Ala Phe Ala Leu Ser Arg Gly Phe Gly Ser Gly Ile
           275                 280                 285
   Ile Thr Ser Asn Ala Pro Met Asp Glu Cys Asp Ala Lys Cys Gln Thr
       290                 295                 300
   Pro Gln Gly Ala Ile Asn Ser Ser Leu Pro Phe Gln Asn Val His Pro
   305                 310                 315                 320
   Val Thr Ile Gly Glu Cys Pro Lys Tyr Val Arg Ser Ala Lys Leu Arg
                   325                 330                 335
   Met Val Thr Gly Leu Arg Asn Ile Pro Ser Ile Gln Ser Arg Gly Leu
               340                 345                 350
   Phe Gly Ala Ile Ala Gly Phe Ile Glu Gly Gly Trp Thr Gly Met Val
           355                 360                 365
   Asp Gly Trp Tyr Gly Tyr His His Gln Asn Glu Gln Gly Ser Gly Tyr
       370                 375                 380
   Ala Ala Asp Gln Lys Ser Thr Gln Asn Ala Ile Asn Gly Ile Thr Asn
   385                 390                 395                 400
   Lys Val Asn Ser Val Ile Glu Lys Met Asn Thr Gln Phe Thr Ala Val
                   405                 410                 415
   Gly Lys Glu Phe Asn Lys Leu Glu Arg Arg Met Glu Asn Leu Asn Lys
               420                 425                 430
   Lys Val Asp Asp Gly Phe Leu Asp Ile Trp Thr Tyr Asn Ala Glu Leu
           435                 440                 445
   Leu Val Leu Leu Glu Asn Glu Arg Thr Leu Asp Phe His Asp Ser Asn
   450 455 460
   Val Lys Asn Leu Tyr Glu Lys Val Lys Ser Gln Leu Lys Asn Asn Ala
   465 470 475 480
   Lys Glu Ile Gly Asn Gly Cys Phe Glu Phe Tyr His Lys Cys Asn Asn
   485 490 495
   Glu Cys Met Glu Ser Val Lys Asn Gly Thr Tyr Asp Tyr Pro Lys Tyr
   500 505 510
   Ser Glu Glu Ser Lys Leu Asn Arg Glu Lys Ile Asp Gly Val Lys Leu
   515 520 525
   Glu Ser Met Gly Val Tyr Gly Gly Ala Gly Pro Pro Pro Pro Val His
   530 535 540
   Leu Pro Pro Pro Val His Leu Pro Pro Pro Val His Leu Pro Pro Pro
   545 550 555 560
   Val His Leu Pro Pro Pro Val His Leu Pro Pro Pro Val His Leu Pro
   565 570 575
   Pro Pro Val His Leu Pro Pro Pro Val His Leu Pro Pro Pro Ser Ala
   580 585 590
   Ala Ser Glu Lys Asp Glu Leu
   595
<210> 17
   <211> 1623
   <212> DNA
   <213> Influenza virus
<400> 17
   gggcccatgg cgaaaaacgt tgcgattttc ggtttattgt tttctcttct tctgttggtt 60
   ccttctcaga tcttcgctga cacaatatgt ataggctacc atgccaacaa ctcaaccgac 120
   actgttgaca cagtacttga gaagaatgtg acagtgacac actctgtcaa cctacttgag 180
   gacagtcaca atggaaaact atgtctacta aaaggaatag ccccactaca attgggtaat 240
   tgcagcgttg ccggatggat cttaggaaac ccagaatgcg aattactgat ttccaaggaa 300
   tcatggtcct acattgtaga aacaccaaat cctgagaatg gaacatgtta cccagggtat 360
   ttcgccgact atgaggaact gagggagcaa ttgagttcag tatcttcatt tgagagattc 420
   gaaatattcc ccaaagaaag ctcatggccc aaccacaccg taaccggagt atcagcatca 480
   tgctcccata atgggaaaag cagtttttac agaaatttgc tatggctgac ggggaagaat 540
   ggtttgtacc caaacctgag caagtcctat gtaaacaaca aagagaaaga agtccttgta 600
   ctatggggtg ttcatcaccc gcctaacata gggaaccaaa gggcactcta tcatacagaa 660
   aatgcttatg tctctgtagt gtcttcacat tatagcagaa gattcacccc agaaatagcc 720
   aaaagaccca aagtaagaga tcaggaagga agaatcaact actactggac tctgctggaa     780
   cctggggata caataatatt tgaggcaaat ggaaatctaa tagcgccatg gtatgctttt     840
   gcactgagta gaggctttgg atcaggaatc atcacctcaa atgcaccaat ggatgaatgt     900
   gatgcgaagt gtcaaacacc tcagggagct ataaacagca gtcttccttt ccagaatgta     960
   cacccagtca caataggaga gtgtccaaag tatgtcagga gtgcaaaatt aaggatggtt    1020
   acaggactaa ggaacatccc atccattcaa tccagaggtt tgtttggagc cattgccggt    1080
   ttcattgaag gggggtggac tggaatggta gatgggtggt atggttatca tcatcagaat    1140
   gagcaaggat ctggctatgc tgcagatcaa aaaagtacac aaaatgccat taacgggatt    1200
   acaaacaagg tcaattctgt aattgagaaa atgaacactc aattcacagc tgtgggcaaa    1260
   gagttcaaca aattggaaag aaggatggaa aacttaaata aaaaagttga tgatgggttt    1320
   ctagacattt ggacatataa tgcagaattg ttggttctac tggaaaatga aaggactttg    1380
   gatttccatg actccaatgt gaagaatctg tatgagaaag taaaaagcca attaaagaat    1440
   aatgccaaag aaataggaaa cgggtgtttt gagttctatc acaagtgtaa caatgaatgc    1500
   atggagagtg tgaaaaatgg tacctatgac tatccaaaat attccgaaga atcaaagtta    1560
   aacagggaga aaattgatgg agtgaaattg gaatcaatgg gagtatacta agagctcagg    1620
   cct                                                                  1623
<210> 18
   <211> 219
   <212> DNA
   <213> Influenza virus
<400> 18
   ggtacctatg actatccaaa atattccgaa gaatcaaagt taaacaggga gaaaattgat      60
   ggagtgaaat tggaatcaat gggagtatac cagattctgg cgatctactc aactgtcgcc     120
   agttccctgg ttcttttggt ctccctgggg gcaatcagct tctggatgtg ttccaatggg     180
   tctttgcagt gtagaatatg catctaagag ctcaggcct                            219
<210> 19
   <211> 123
   <212> DNA
   <213> Artificial
<220>
   <223> ER-retained SEKDEL gene fragment
<400> 19
   ggtacctatg actatccaaa atattccgaa gaatcaaagt taaacaggga gaaaattgat 60
   ggagtgaaat tggaatcaat gggagtatac tctgagaagg atgaacttta agagctcagg 120
   cct 123
<210> 20
   <211> 117
   <212> DNA
   <213> Artificial
<220>
   <223> ER-retained HDEL gene fragment
<400> 20
   ggtacctatg actatccaaa atattccgaa gaatcaaagt taaacaggga gaaaattgat 60
   ggagtgaaat tggaatcaat gggagtatac cacgatgaac tttaagagct caggcct 117
<210> 21
   <211> 207
   <212> DNA
   <213> Artificial
<220>
   <223> GCN4-pII gene fragment
<400> 21
   ggtacctatg actatccaaa atattccgaa gaatcaaagt taaacaggga gaaaattgat 60
   ggagtgaaat tggaatcaat gggagtatac ctcaaacaaa tcgaagacaa gatcgaagag 120
   atcctctcga aaatctacca catcgaaaac gagattgcca ggatcaagaa gctcataggc 180
   gagtcagcag cctaagagct caggcct 207
<210> 22
   <211> 225
   <212> DNA
   <213> Artificial
<220>
   <223> ER-retained GCN4-pII gene fragment
<400> 22
   ggtacctatg actatccaaa atattccgaa gaatcaaagt taaacaggga gaaaattgat 60
   ggagtgaaat tggaatcaat gggagtatac ctcaaacaaa tcgaagacaa gatcgaagag 120
   atcctctcga aaatctacca catcgaaaac gagattgcca ggatcaagaa gctcataggc 180
   gagtcagcag cctctgagaa ggatgaactt taagagctca ggcct 225
<210> 23
   <211> 279
   <212> DNA
   <213> Zea maize
<400> 23
   ggtacctatg actatccaaa atattccgaa gaatcaaagt taaacaggga gaaaattgat     60
   ggagtgaaat tggaatcaat gggagtatac ggaggcgctg gcccaccgcc agttcatcta    120
   ccgcctccgg tgcatctgcc acctccggtt cacctgccac ctccggtgca tctcccaccg    180
   ccagtccacc tgccgcctcc ggtccacctg ccaccgcctg tccatgtgcc gccaccggtt    240
   catctgccgc ctccatcagc agcctaagag ctcaggcct                           279
<210> 24
   <211> 297
   <212> DNA
   <213> Artificial
<220>
   <223> ER-retained gene fragment
<400> 24
   ggtacctatg actatccaaa atattccgaa gaatcaaagt taaacaggga gaaaattgat     60
   ggagtgaaat tggaatcaat gggagtatac ggaggcgctg gcccaccgcc agttcatcta    120
   ccgcctccgg tgcatctgcc acctccggtt cacctgccac ctccggtgca tctcccaccg    180
   ccagtccacc tgccgcctcc ggtccacctg ccaccgcctg tccatgtgcc gccaccggtt    240
   catctgccgc ctccatcagc agcctctgag aaggatgaac tttaagagct caggcct      297

## Description

### FIELD OF THE INVENTION

The present invention relates to the production of soluble recombinant influenza antigens. More specifically, the present invention is directed to the production of soluble recombinant influenza antigens that retain immunogenicity.

### BACKGROUND OF THE INVENTION

Influenza is the leading cause of death in humans due to a respiratory virus. Common symptoms include fever, sore throat, shortness of breath, and muscle soreness, among others. During flu season, influenza viruses infect 10-20% of the population worldwide, leading to 250-500,000 deaths annually.

Influenza viruses are classified into types A, B, or C, based on the nucleoproteins and matrix protein antigens present. Influenza type A viruses may be further divided into subtypes according to the combination of hemagglutinin (HA) and neuraminidase (NA) surface glycoproteins presented. HA governs the ability of the virus to bind to and penetrate the host cell. NA removes terminal sialic acid residues from glycan chains on host cell and viral surface proteins, which prevents viral aggregation and facilitates virus mobility. Currently, 16 HA (H1-H16) and 9 NA (N1-N9) subtypes are recognized. Each type A influenza virus presents one type of HA and one type of NA glycoprotein. Generally, each subtype exhibits species specificity; for example, all HA and NA subtypes are known to infect birds, while only subtypes H1, H2, H3, H5, N1 and N2 have been shown to infect humans. Influenza viruses comprising H5 and H7 are considered the most highly pathogenic forms of influenza A viruses, and are most likely to cause future pandemics.

Influenza pandemics are usually caused by highly transmittable and virulent influenza viruses, and can lead to elevated levels of illness and death globally. The emergence of new influenza A subtypes resulted in 4 major pandemics in the 20^{th} century. The Spanish flu, caused by an H1N1 virus, in 1918-1919 led to the deaths of over 50 million people worldwide between 1917 and 1920. The risk of the emergence of a new subtype, or of the transmission to humans of a subtype endemic in animals, is always present. Of particular concern is a highly virulent form of avian influenza (also called "bird flu"), outbreaks of which have been reported in several countries around the world. In many cases, this bird flu can result in mortality rates approaching 100% within 48 hours. The spread of the avian influenza virus (H5N1), first identified in Hong Kong in 1997, to other Asian countries and Europe has been postulated to be linked to the migratory patterns of wild birds.

There is increasing concern that the virus may become highly infectious for humans. The major problem for human health is the fact that influenza viruses are antigenically unstable, that is, they mutate rapidly. Should the avian influenza virus come into contact with human viruses, genetic reassortment of the avian virus could result in a highly pathogenic influenza virus that could causes severe disease or death in humans. Furthermore, such mutation could result in an influenza virus that is easily transmitted in humans.

The current method of combating influenza in humans is by annual vaccination. Each year, the World Health Organization selects 3 viral strains for inclusion in the annual influenza vaccine, which is produced in fertilized eggs. However, the number of vaccine doses produced each year is not sufficient to vaccinate the world's population. For example, Canada and the United-States obtain enough vaccines doses to immunize about one third of their population, while only 17% of the population of the European Union can be vaccinated. It is evident that current worldwide production of influenza vaccine would be insufficient in the face of a worldwide flu pandemic. Therefore, governments and private industry alike have turned their attention to the productions of effective influenza vaccines.

As previously mentioned, the current method of obtaining influenza virus vaccines is by production in fertilized eggs. The virus is cultured in fertilized eggs, followed by inactivation of the virus and purification of viral glycoproteins. While this method maintains the antigenic epitope and post-translational modifications, there are a number of drawbacks including the risk of contamination due to the use of whole virus and variable yields depending on virus strain. Sub-optimal levels of protection may result from genetic heterogeneity in the virus due to its introduction into eggs. Other disadvantages includes extensive planning for obtaining eggs, contamination risks due to chemicals used in purification, and long production times. Also, persons hypersensitive to egg proteins may not be eligible candidates for receiving the vaccine.

To avoid the use of eggs, influenza viruses have also been produced in mammalian cell culture, for example in MDCK or PERC.6 cells, or the like. Another approach is reverse genetics, in which viruses are produced by cell transformation with viral genes. These methods, however, also requires the use of whole virus as well as elaborate methods and specific culture environments.

The use of viral DNA as a vaccine has been explored. In this technology, protection is obtained by expression of viral antigens in human cells; the antigens are then recognized as foreign antigen, which leads to an specific antibody response. However, there exists the risk of oncogene activation from introduction of DNA into determinant portion of human cell genome - a significant drawback.

Vaccines comprising recombinant viral antigens expressed in viral DNA transformed insect or plant cells have also been prepared by Dow Agroscience (see, for example WO 2004/098530). While the risk associated with the use of live virus is avoided and the production process is shorter, protein conformation and post-translational modifications are affected. The scale-up and purification steps are also relatively complex as the antigens are associated with cellular membranes. In addition, the dose of baculovirus-recombinant HA required for effective immunization in animals is 10-fold higher than that of natural HA produced in fertilized eggs. In both cases, the levels of viral antigen expression are low.

In an effort to avoid the difficulties associated with purification of membrane proteins, Huang et al (2001, Vaccine, 19:2163-2171) replaced the transmembrane domain and the cytoplasmic tail of the measles HA with an ER retention signal. The resulting HA protein was produced in tobacco plant cells for the development of oral vaccine (edible vaccine). The HA expressed is not as strongly retained in the ER as it is with the transmembrane domain, thus simplifying the purification procedure. However, the natural trimeric form of HA cannot be formed in those conditions, which can affect the immunogenicity of the recombinant protein.

Saelens et al (1999, Eur. J. Biochm, 260:166-175) expressed an HA gene lacking the transmembrane domain in yeast (*Pichia pastoris*)*,* leading to the secretion of monomeric HA. This form, however, was less immunogenic than the trimeric HA. The publication of Wei et al "Comparative Efficacy of Neutralizing Antibodies Elicited by Recombinant Hemagglutinin Proteins from Avian H5N1 Influenza Virus", Journal of Virology, July 2008, p. 6200-6208 analyzes various forms of rHA protein for their potential efficacy as vaccines. WO 02/074795 relates to a chimeric protein comprising an antigen and an oligomerisation domain. WO 2008/054540 provides polynucleotides and polypeptides capable of enhancing the immune response of a human in need of protection against influenza virus infection. WO 2009/076778 refers to a method for synthesizing influenza virus-like particles (VLPs) within a plant or a portion of a plant. Mett et al refers in Journal Compilation 2008, 2, p. 33-40 to a plant-produced influenza subunit vaccine protects ferrets against virus challenge. In Vaccine 26(2008), p. 2930-2934 Shoji et al refers to plant-expressed HA as a seasonal influenza vaccine candidate. Huang et al study Vaccine 19(2001), p. 2163-2171 plant-derived measles virus hemagglutinin protection which includes neutralizing antibodies in mice. The Switch Between Two-, Three- and Four-Stranded Coiled Coils in GCN4 Leucine Zipper Mutants is studied in Science, Vol. 262, 26 November 1993, p. 1401-1407 by Harbury et al.

In order to protect the world population from influenza and to stave off future pandemics, vaccine manufacturers will need to develop effective, rapid methods producing vaccine doses. The current use of fertilized eggs to produce vaccines is insufficient and involves a lengthy process. Recombinant technologies offer promising approaches to the production of influenza antigens. However, the production of hemagglutinin has been limited to membrane-associated protein, which involves complex extraction processes with low yields, or to poorly-immunogenic soluble proteins.

### SUMMARY OF THE INVENTION

The present invention relates to the production of soluble recombinant influenza antigens. More specifically, the present invention is directed to the production of soluble recombinant influenza antigens that retain trimeric assembly and immunogenicity.

It is an object of the present invention to provide soluble recombinant influenza antigens.

The present invention provides a recombinant hemagglutinin (rHA), comprising a hemagglutinin domain and an oligomerization domain, a signal peptide and endoplasmic reticulum (ER) as defined in claim 1. The rHA is produced as a chimeric soluble homotrimer.

The present invention also provides a nucleotide sequence encoding the rHA as just described above.

The present invention further provides a nucleic acid sequence comprising a) a nucleotide sequence encoding a hemagglutinin domain; and b) a nucleotide sequence encoding an oligomerization domain. The nucleic acid encodes a soluble rHA that forms a homotrimer. The nucleic acid further comprises a nucleotide sequence encoding a signal peptide and an endoplasmic reticulum (ER) retention signal.

The present invention also provides a vector comprising the nucleotide as described above.

The invention further provides a host cell expressing the rHA as described above, a host cell transformed with the nucleotide as just described above, or a host cell transformed with the vector as just described above.

It is also provided by the present invention, a method of producing a recombinant rHA protein. The method comprises providing a host cell with a vector comprising: a) a nucleotide sequence encoding a hemagglutinin domain, wherein the nucleic acid encodes a soluble rHA that forms a homotrimer; and b) a nucleotide sequence encoding an oligomerization domain, then expressing the rHA.

The present invention further provides a method of expressing a recombinant hemagglutinin (rHA) within a plant. In a first step, a vector comprising a nucleotide sequence encoding a hemagglutinin domain, wherein the nucleic acid encodes a soluble rHA that forms a homotrimer and a nucleotide sequence encoding an oligomerization domain, is introduced into a plant. In the step of introducing (step a), the nucleic acid may be introduced in the plant in a transient manner, or the nucleic acid is introduced in the plant so that it is stable.

The present invention also provides a method of producing a recombinant hemagglutinin (rHA) in a plant, comprising: a) introducing a nucleic acid sequence into the plant, or portion thereof, the nucleic acid sequence comprising a regulatory region operatively linked to a nucleotide sequence encoding a hemagglutinin domain and an oligomerization domain, wherein the nucleic acid encodes a soluble rHA that forms a homotrimer; and b) growing the transgenic plant, thereby producing the rHA. In the step of introducing (step a), the nucleic acid may be introduced in the plant in a transient manner, or the nucleic acid is introduced in the plant so that it is stable.

rHA is a very complex molecule to produce. Expression levels and yields of recombinant HA from current production systems are low; and consequently the production costs are high. This is mostly due to the complex trimeric structure of the protein, which must undergo a complex process for assembly during its synthesis. Furthermore, HA is a large protein that has a transmembrane domain, and is highly glycosylated. Producing a soluble form of HA would allow production at higher levels and decrease the complexity of the purification process. This would have an important impact on production costs. Replacing the transmembrane domain with soluble α-helices or other secondary structure suitable to stabilize HA structurally compatible with the coiled-coil core of the ectodomain of the HA protein is shown to yield stable, soluble HA trimers. Such recombinant proteins can be used to enrich current influenza vaccines, or in the preparation of new vaccines.

### BRIEF DESCRIPTION OF THE DRAWINGS

These and other features of the invention will become more apparent from the following description in which reference is made to the appended drawings wherein:

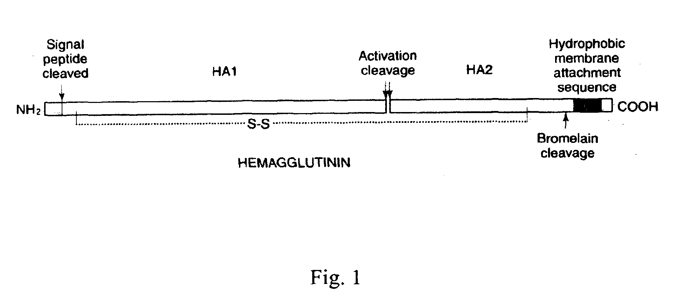

**FIGURE 1** shows a schematic of the domains of the naturally occurring hemagglutinin (HA) protein.

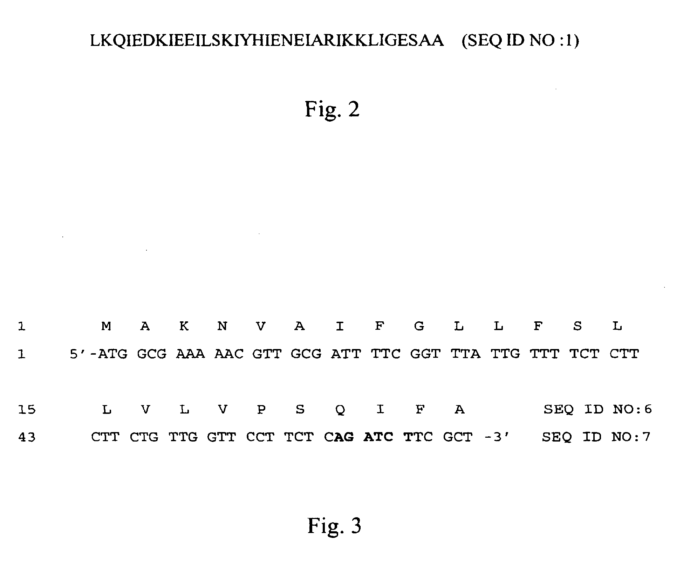

**FIGURE 2** shows the amino acid sequence of the GCN4-pII peptide (SEQ ID NO:1).
**FIGURE 3** shows the amino acid and nucleotide sequences of PDI (SEQ ID NOs:6 and 7; (Genbank Accession Z11499), an alfalfa signal peptide. The PDI signal peptide is homologous to mouse ERp59. The *Bgl*II restriction site is indicated in bold.

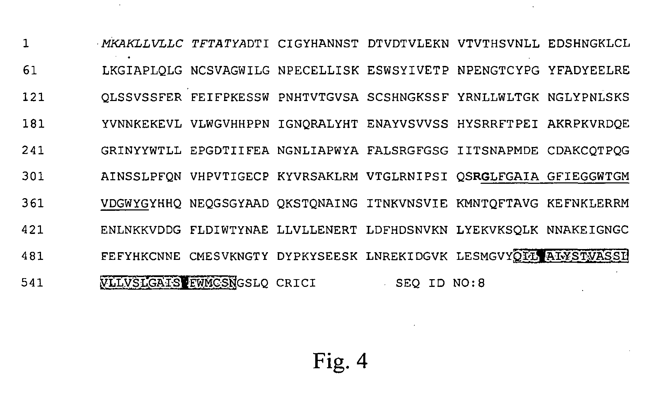

**FIGURE 4** shows the amino acid sequence (SEQ ID NO:8) of HA from influenza strain A/New Caledonia/20/99 (H1N1) (Genbank Accession AY289929; Primary accession UniProt KB/TrEMBL: Q6WG00). The rHA signal peptide is shown in italics. The cleavage site of HA0 is indicated in bold and the fusion peptide is underlined. The transmembrane domain is shown in gray background.

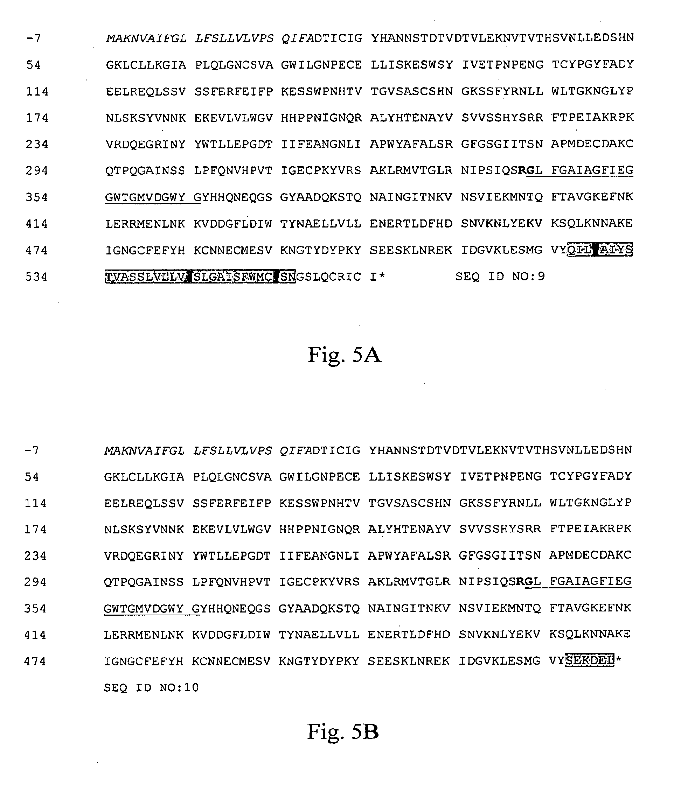

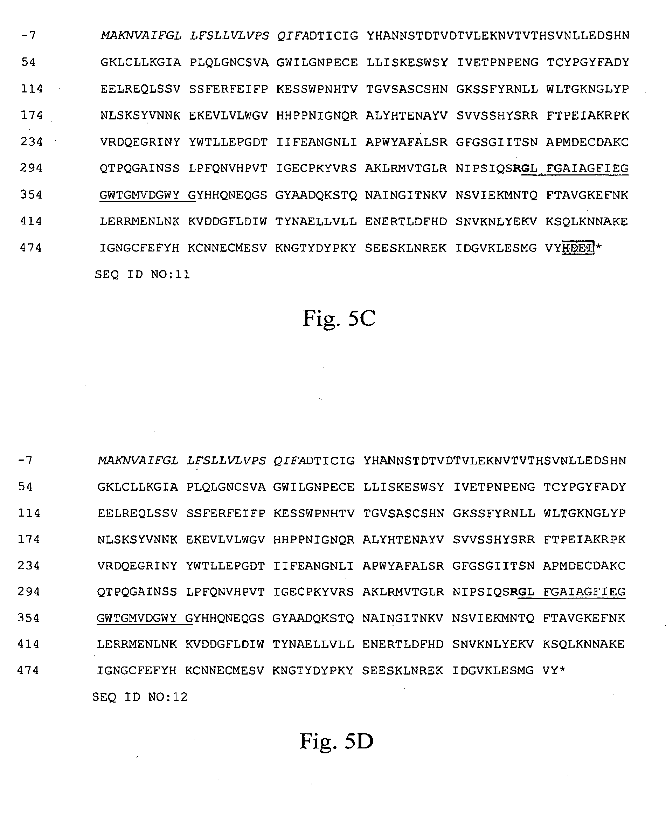

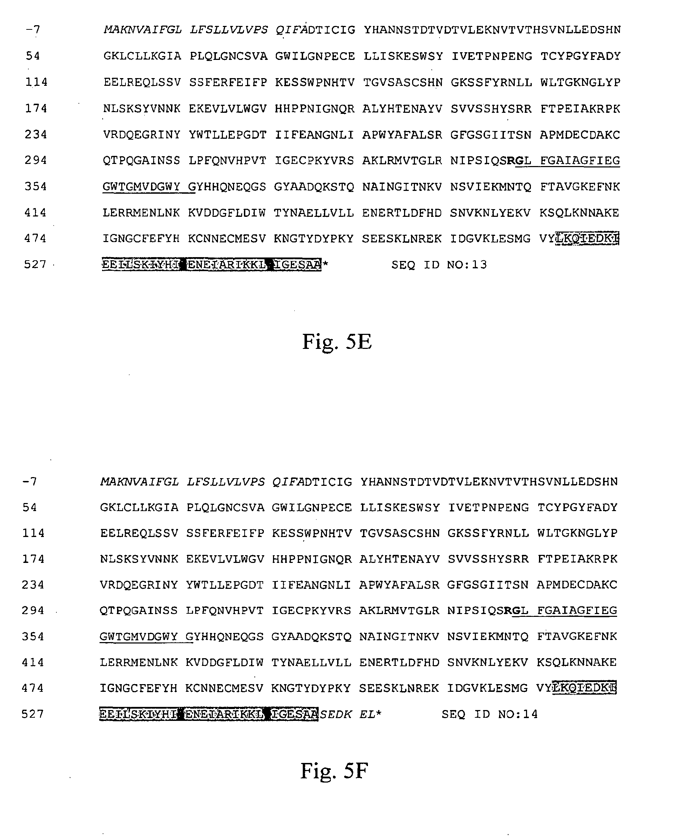

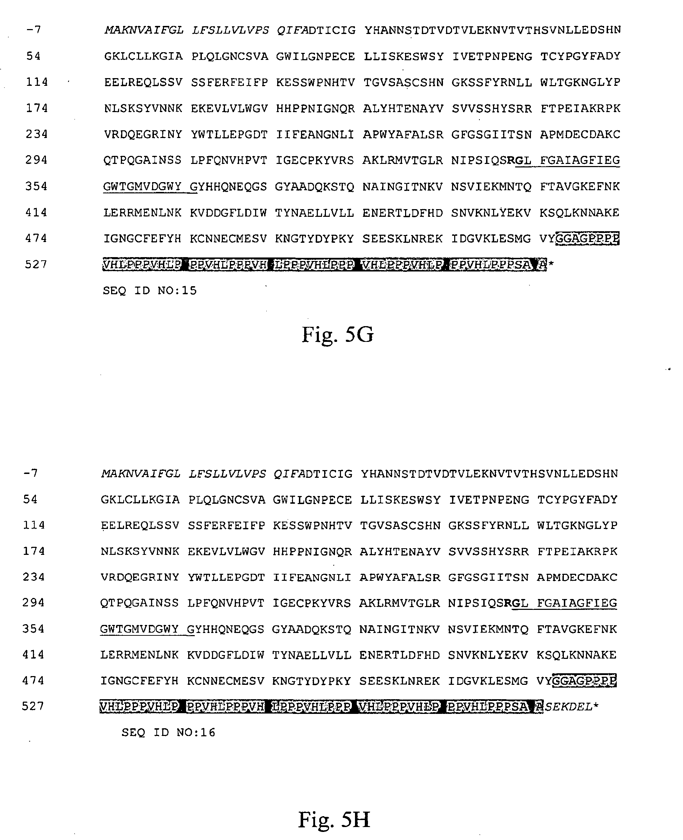

**FIGURE 5** shows the amino acid sequences of various rHA constructs according to the present invention. The amino acid numbering has been adjusted according to the original amino acid numbering of HA. The PDI signal peptide is indicated in italics, the HA₀ cleavage site is shown in bold, the fusion peptide is underlined, and the stop codon is represented by *. Figure 5A is the amino acid sequence (SEQ ID NO:9) of full length rHA comprising the PDI signal peptide and the tramsmembrane domain and cytoplasmic tail. The transmembrane domain is shown in gray background. Figure 5B is the amino acid sequence (SEQ ID NO:10) of ER-retained rHA using the SEKDEL retention signal. The retention signal is shown in gray background. Figure 5C is the amino acid sequence (SEQ ID NO:11) of ER-retained rHA using the HDEL retention signal. The retention signal is shown in gray background. Figure 5D is the amino acid sequence (SEQ ID NO:12) of soluble rHA without the transmembrane domain. Figure 5E is the amino acid sequence (SEQ ID NO:13) of soluble trimeric rHA using the GCN4-pII trimeric peptide. The GCN4-pII peptide is shown in gray background. Figure 5F is the amino acid sequence (SEQ ID NO:14) of soluble trimeric rHA using the GCN4-pII trimeric peptide and retained in the ER. The GCN4-pII peptide is shown in gray background and the SKDEL retention signal is shown in italics. Figure 5G is the amino acid sequence (SEQ ID NO:15) of soluble trimeric rHA using the PRD trimeric peptide. The PRD peptide is shown in gray background. Figure 5H is the amino acid sequence (SEQ ID NO:16) of soluble trimeric rHA using the PRD trimeric peptide, and retained in the ER. The PRD peptide is shown in gray background and the retention signal is shown in italics.

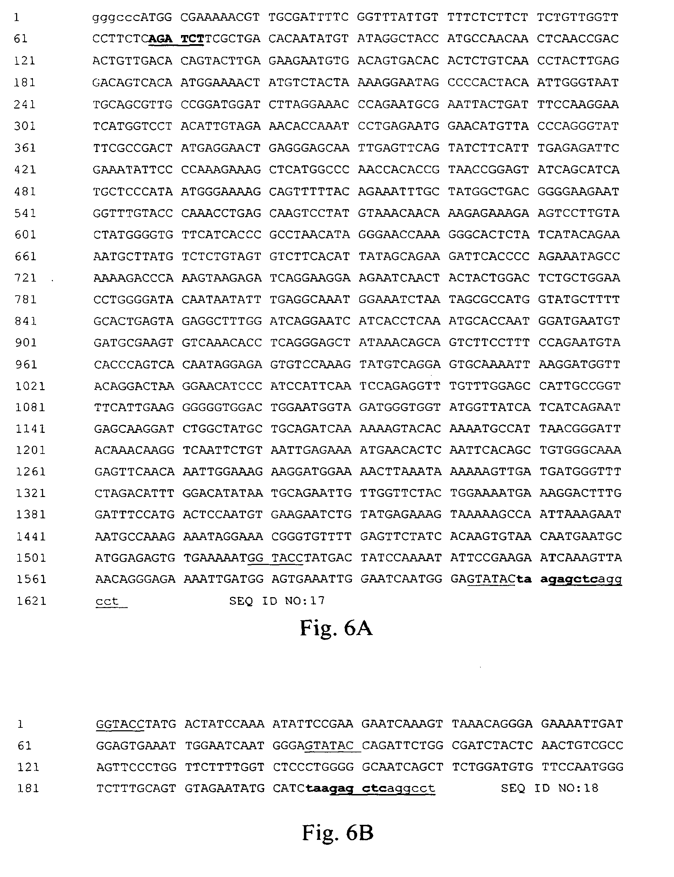

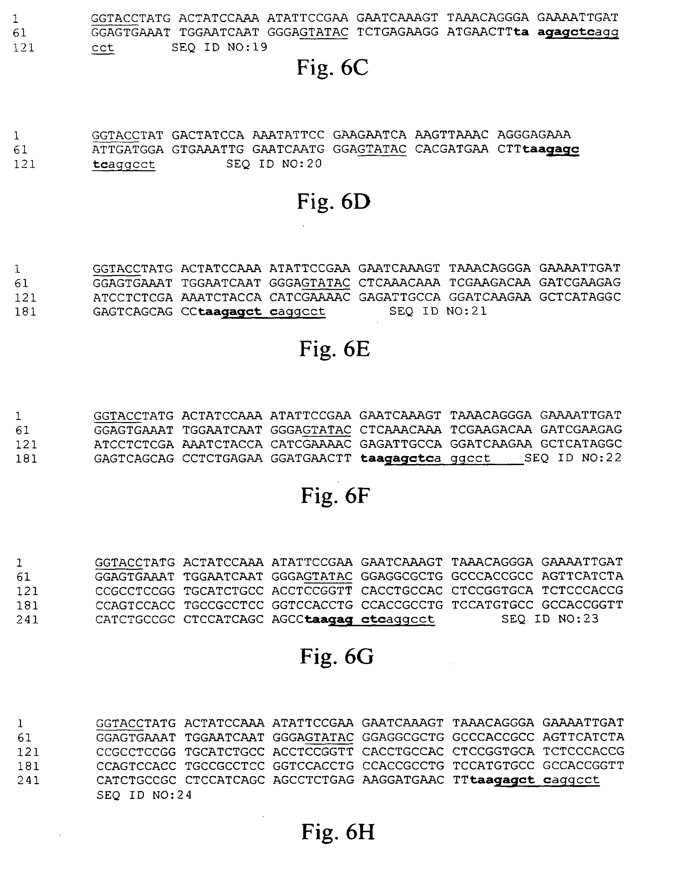

**FIGURE 6** shows the nucleotide sequences of various fragments according to the present invention. The non-coding sequence is presented in small capitals and useful restriction sites are underlined. Figure 6A shows the nucleotide sequence (SEQ ID NO:17) of the HA₀ gene fragment. Figure 6B shows the nucleotide sequence (SEQ ID NO:18) of the transmembrane domain and the cytoplasmic tail gene fragment. Figure 6C shows the nucleotide sequence (SEQ ID NO:19) of the ER-retained SEKDEL gene fragment. Figure 6D shows the nucleotide sequence (SEQ ID NO:20) of the ER-retained HDEL gene fragment. Figure 6E shows the nucleotide sequence (SEQ ID NO:21) of the GCN4-pII gene fragment. Figure 6F shows the nucleotide sequence (SEQ ID NO:22) of the ER-retained GCN4-pII gene fragment. Figure 6G shows the nucleotide sequence (SEQ ID NO:23) of the PRD gene fragment. Figure 6H shows the nucleotide sequence (SEQ ID NO:24) of the ER-retained PRD gene fragment.

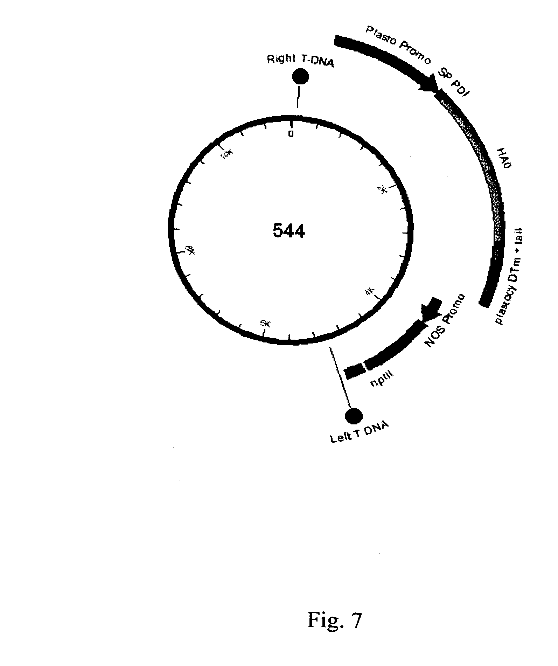

**FIGURE 7** is a schematic diagram of the rHA transfer DNA (t-DNA) in a pCAMBIA binary plasmid, in accordance with one embodiment of the present invention.

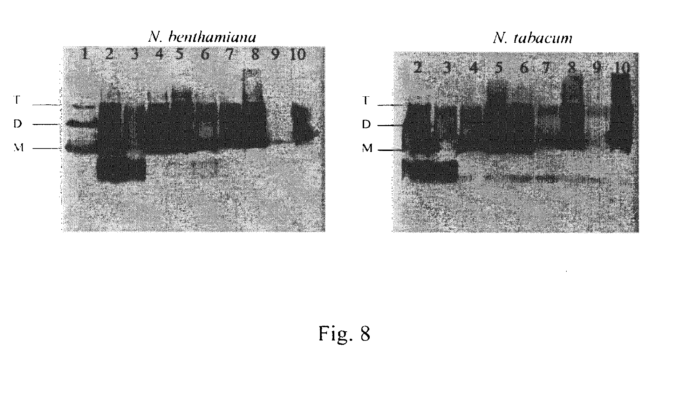

**FIGURE 8** is a Western blot showing the immunodetection of rHA expression in tobacco. Lanes: 1) Pure rHA standard (1ng); 2) 1 ng of standard rHA spiked into 10 µg of plant extract; 3) 10 µg of plant extract; 4) 10 µg of protein extract from biomass expressing construct #540; 5) 10 µg of protein extract from biomass expressing construct #541; 6) 10 µg of protein extract from biomass expressing construct #542; 7) 10 µg of protein extract from biomass expressing construct #544; 8) 10 µg of protein extract from biomass expressing construct #545; 9) 10 µg of protein extract from biomass expressing construct #546; and 10) 10 µg of protein extract from biomass expressing construct #547.

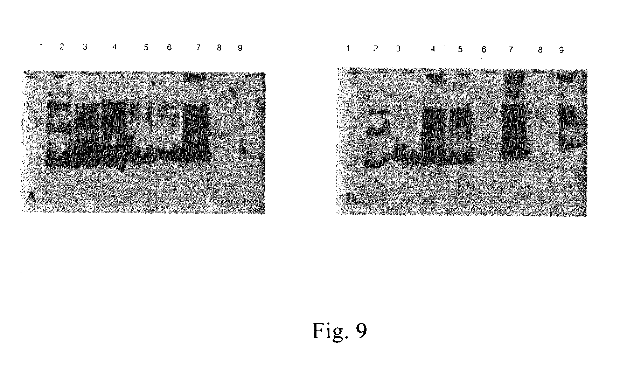

**FIGURE 9** is a Western blot showing the immunodetection of rHA expression in tobacco, with 5 µg pf extract. Figure 9A shows results from *N*. *benthamiana,* while Figure 9B shows results from *N*. *tabacum.* Lanes: 1) 5 µg of plant extracts for panels B, D and A,C respectively; 2) 1 ng of standard rHA spiked into 2 and 5 µg of plant extract for panels B, D and A,C, respectively; 3) Extract from biomass expressing construct #540; 4) Extract from biomass expressing construct #541; 5) Extract from biomass expressing construct #542; 6) Extract from biomass expressing construct #544; 7) Extract from biomass expressing construct #545; 8) Extract from biomass expressing construct #546; and 9) Extract from biomass expressing construct #547.

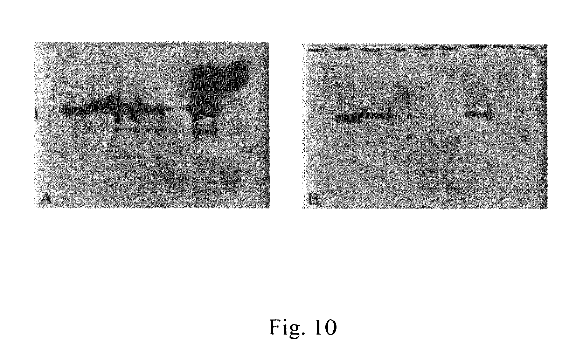

**FIGURE 10** is a Western blot showing the immunodetection of rHA expression in tobacco, with 5 µg pf extract, under reducing conditions. Figure 10A shows results from *N. benthamiana,* while Figure 10B shows results from *N*. *tabacum.* Lanes: 1) 5 µg of plant extract; 2) 1 ng of standard rHA spiked into 5 µg of plant extract; 3) Extract from biomass expressing construct #540; 4) Extract from biomass expressing construct #541; 5) Extract from biomass expressing construct #542; 6) Extract from biomass expressing construct #544; 7) Extract from biomass expressing construct #545; 8) Extract from biomass expressing construct #546; and 9) Extract from biomass expressing construct #547.

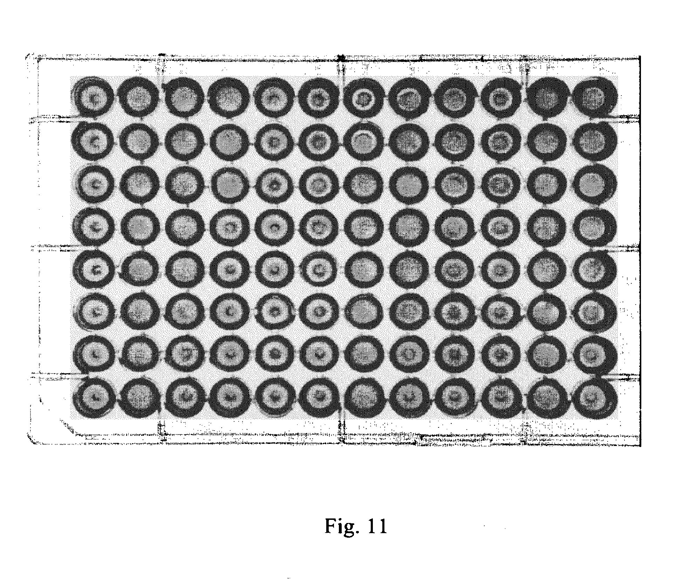

**FIGURE 11** shows a plate with results of a hemagglutination assay. Row 1: PBS (Negative control); Row 2: PBS + 1000 ng HA (PSC); Row 3: PBS + 100 ng HA (PSC); Row 4: PBS + 10 ng of HA (PSC); Row 5: PBS + 1 ng of HA (PSC); Row 6: Non-transformed plant extract; Row 7: Non-transformed plant extcact+ 1000 ng HA (PSC); Row 8: Non-transformed plant extract+ 100 ng HA (PSC); Row 9: Non-transformed plant extract + 10 ng HA (PSC); Row 10: Non-transformed plant extract + 1 ng HA (PSC); Row 11: Plant extract expressing construct 540 (transmembranar rHA); and Row 12: Plant extract expressing construct 544 (soluble rHA fused to GCN4).

### DESCRIPTION OF PREFERRED EMBODIMENT

The present invention relates to the production of soluble recombinant influenza antigens. More specifically, the present invention is directed to the production of soluble recombinant influenza antigens that retain immunogenicity.

The following description is of a preferred embodiment.

The present invention provides a recombinant hemagglutinin (rHA) comprising a hemagglutinin domain and an oligomerization domain. The recombinant protein is produced as a soluble homotrimer. The rHA also comprises a signal peptide and an endoplasmic reticulum (ER) retention signal.

Influenza is caused by influenza viruses, which are classified into types A, B, or C. Type A and B viruses are most often associated with epidemics. Influenza type A viruses is further divided into subtypes according to the combination of hemagglutinin (HA) and neuraminidase (NA) surface glycoproteins presented. Currently, 16 HA (H1-H16) and 9 NA (N1-N9) subtypes are recognized. Each type A influenza virus presents one type of HA and one type of NA glycoprotein.

By the term recombinant hemagglutinin, also refered to as "recombinant HA" and "rHA", it is meant a hemagglutinin protein that is produced by recombinant techniques, which are well known by a person of skill in the art. Hemagglutinin (HA) is an viral surface protein found on type A influenza viruses. To date, sixteen HA subtypes (H1-H16) have been identified. HA is responsible for binding of the virus to sialic acid residues of carbohydrate moieties on the surface of the infected host cell. Following endocytosis of the virus by the cell, the HA protein undergoes drastic conformational changes which initiate the fusion of viral and cellular membranes and virus entry into the cell.

HA is a homotrimeric membrane type I glycoprotein, generally comprising a signal peptide, a HA₀ domain, a membrane-spaning anchor site at the C-terminus and a small cytoplasmic tail (Figure 1). The term "homotrimer" or "homotrimeric" indicates that an oligomer is formed by three HA protein molecules. HA protein is synthesized as a 75 kDa monomeric precursor protein (HA₀), which assembles at the surface into an elongated trimeric protein. Before triinerization occurs, the precursor protein HA0 is cleaved at a conserved activation cleavage site (also refered to as fusion peptide) into 2 polypeptide chains, HA1 (328 amino acids) and HA2 (221 amino acids), linked by a disulfide bond. Although this step is central for virus infectivity, it is not essential for the trimerization of the protein. Insertion of HA within the endoplasmic reticulum (ER) membrane of the host cell, signal peptide cleavage and protein glycosylation are co-translational events. Correct refolding of HA requires glycosylation of the protein and formation of 6 intra-chain disulfide bonds. The HA trimer assembles within the cis- and trans-Golgi complex, the transmembrane domain playing a role in the trimerization process. The crystal structures of bromelain-treated HA proteins, which lack the transmembrane domain, have shown a highly conserved structure amongst influenza strains (Russell *et al.* 2004). It has also been established that HA undergoes major conformational changes during the infection process, which requires the precursor HA₀ to be cleaved into the 2 polypeptide chains HA1 and HA2.

The recombinant HA of the present invention is a subtype selected from the group H1, H2, H3, H4, H5, H6, H7, H8, H9, H10, H11, H12, H13, H14, H15, or H16. Furthermore, the rHA may be based on the sequence of a hemagglutinin that is isolated from emerging influenza viruses.

The rHA of the present invention is a chimeric protein construct comprising a hemagglutinin domain and an oligomerization domain. The term "hemagglutinin domain" refers to an amino acid sequence comprising either the HA₀ domain, or the HA1 and HA2 domains. In other words, the rHA protein may be processed (i.e., comprises HA1 and HA2 domains), or may be unprocessed (i.e., comprises the HA₀ domain). The hemagglutinin domain does not include the signal peptide, transmembrane domain, or the cytoplasmic tail found in the naturally occurring protein. The "oligomerization domain", also referred to as "trimeric peptide", refers to a domain that promotes the oligomerization of the rHA protein. The oligomerization domains include the GCN4-pII peptide (Harbury et al, 1993, Science, 262:1401-7), and the proline-rich domain (PRD) of maize γ-zein.

The oligomerization domain may be the GCN4-pII peptide, a variant of the GCN4 yeast leucine zipper. This GCN4 mutant bears Ile residues at both a and d positions (pII), present at every 7 amino acids on the alpha-helix, leading to a high propencity for trimerization. The melting temperature (Tm) of the trimer is >100°C, conferring a high intrinsic stability to the oligomer (Harbour et al.). The amino acid sequence of GCN4-pII is shown in Figure 2. GCN4-pII is well-suited for use as the oligomerization domain; the 29 amino acid sequence of GCN4-pII is placed at the C-terminal end of the HA domain sequence, essentially replacing the 26 amino acid transmembrane domain. If desired, additional amino acids may be placed at the C-terminal end of the rHA such that the recombinant structure does not terminate by an α-helix. For example, Ser-Ala-Ala amino acid residues may be added at the C-terminal end of GCN4-pII.

The oligomerization domain may be the PRD of maize γ-zein, also referred to herein as "PRD". Maize gamma-γ-Zein is known to store stacks in protein bodies once inside the ER. The synthetic PRD peptide adopts an amphipathic polyproline II conformation, which assembles itself as trimers (Kogan et al, 2002, Biophysical J., 83:1194-1204). In its natural form, the PRD comprises 8 repeats of the peptide PPPVHL (SEQ ID NO:2). The PRD peptide of maize gamma-γ-Zein is also placed at the C-terminus of the HA domain, replacing the transmembrane anchor. As the natural form of PRD is quite long, it is also within the scope of the present invention to provide the PRD in varying peptide lengths (4, 6 or 8 peptide repeats, i.e., 24, 36, or 48 amino acids in length). If desired, an amino acid linker may be placed between the HA domain and the PRD in order to permit the orientation of the PRD peptide chain towards a polyproline left helix. Any suitable peptide linker known in the art may be used. For example, a tetrapeptide such as Gly-Gly-Ala-Gly (SEQ ID NO:3) may be used. Also if desired, additional amino acids may be placed at the C-terminal end of the rHA such that the recombinant structure does not terminate by an α-helix. For example, Ser-Ala-Ala amino acid residues may be added at the C-terminal end of PRD.

The rHA according to the present invention further comprises a signal peptide. The signal peptide may be any suitable peptide known in the art, to direct the recombinant protein to the desired cell compartment or membrane. For example, the signal peptide found in natural HA may be used, which directs HA the ER. In another example, the signal peptide may be PDI, the alfalfa signal peptide. The amino acid and nucleotide sequences of PDI are shown in Figure 3. Advantageously, the PDI signal peptide has a *bg*/II restriction site, which may be useful for cloning.

The rHA as described above also further comprises an endoplasmic reticulum (ER) retention signal. Any suitable ER retention signal known by a person of skill in the art may be used. For example, the Ser-Glu-Lys-Asp-Glu-Leu (SEKDEL; SEQ ID NO:4) or the His-Asp-Glu-Leu (HDEL; SEQ ID NO:5) ER retention signals may be used. The chosen ER retention signal may be at the C-terminal end of the rHA protein sequence. Advantageously, ER-retention of a recombinant protein in plants has been shown in several cases to improve the expression level by 2- to 10-fold (Schillberg et al, 2003, Cell Mol. Life Sci. 60:443-445). Without wishing to be bound by theory, the ER retention signals may allow back and forth movement of proteins between the ER and the Golgi complex, which allows trimerization to occur.

The term "soluble" indicates that the rHA is produced in the host cell in a soluble form. As described above, the conversion of recombinant HA to a soluble form arises from the replacement of the transmembrane hydrophobic domain by a soluble α-helices that are structurally compatible with the HA domain. Expressing the rHA in a soluble form may increase yields (higher expression levels) and decrease the complexity of purification, therefore lowering the production costs.

The present invention also provides a nucleic acid encoding the rHA as described above. The nucleic acid is a chimeric construct comprising a nucleotide sequence encoding a hemagglutinin domain (HA₀) and a nucleotide sequence encoding an oligomerization domain. The nucleic acid encoding the rHA also comprises a nucleotide sequence encoding a signal peptide and a nucleotide sequence encoding an ER retention signal.

The present invention is further directed to a chimeric gene construct comprising a nucleic acid encoding rHA, as described above, operatively linked to a regulatory element. By "regulatory element" or "regulatory region", it is meant a portion of nucleic acid typically, but not always, upstream of a gene, and may be comprised of either DNA or RNA, or both DNA and RNA. Regulatory elements may include those which are capable of mediating organ specificity, or controlling developmental or temporal gene activation. Furthermore, "regulatory element" includes promoter elements, core promoter elements, elements that are inducible in response to an external stimulus, elements that are activated constitutively, or elements that decrease or increase promoter activity such as negative regulatory elements or transcriptional enhancers, respectively. By a nucleotide sequence exhibiting regulatory element activity it is meant that the nucleotide sequence when operatively linked with a coding sequence of interest functions as a promoter, a core promoter, a constitutive regulatory element, a negative element or silencer (i.e. elements that decrease promoter activity), or a transcriptional or translational enhancer.

By "operatively linked" it is meant that the particular sequences, for example a regulatory element and a coding region of interest, interact either directly or indirectly to carry out an intended function, such as mediation or modulation of gene expression. The interaction of operatively linked sequences may, for example, be mediated by proteins that interact with the operatively linked sequences.

Regulatory elements as used herein, also includes elements that are active following transcription initiation or transcription, for example, regulatory elements that modulate gene expression such as translational and transcriptional enhancers, translational and transcriptional repressors, and mRNA stability or instability determinants. In the context of this disclosure, the term "regulatory element" also refers to a sequence of DNA, usually, but not always, upstream (5') to the coding sequence of a structural gene, which includes sequences which control the expression of the coding region by providing the recognition for RNA polymerase and/or other factors required for transcription to start at a particular site. An example of a regulatory element that provides for the recognition for RNA polymerase or other transcriptional factors to ensure initiation at a particular site is a promoter element. A promoter element comprises a core promoter element, responsible for the initiation of transcription, as well as other regulatory elements (as listed above) that modify gene expression. It is to be understood that nucleotide sequences, located within introns, or 3' of the coding region sequence may also contribute to the regulation of expression of a coding region of interest. A regulatory element may also include those elements located downstream (3') to the site of transcription initiation, or within transcribed regions, or both. In the context of the present invention a post-transcriptional regulatory element may include elements that are active following transcription initiation, for example translational and transcriptional enhancers, translational and transcriptional repressors, and mRNA stability determinants.

The regulatory elements, or fragments thereof, may be operatively associated (operatively linked) with heterologous regulatory elements or promoters in order to modulate the activity of the heterologous regulatory element. Such modulation includes enhancing or repressing transcriptional activity of the heterologous regulatory element, modulating post-transcriptional events, or both enhancing/repressing transcriptional activity of the heterologous regulatory element and modulating post-transcriptional events. For example, one or more regulatory elements, or fragments thereof, may be operatively associated with constitutive, inducible, tissue specific promoters or fragment thereof, or fragments of regulatory elements, for example, to TATA or GC sequences may be operatively associated with the regulatory elements of the present invention, to modulate the activity of such promoters within plant, insect, fungi, bacterial, yeast, or animal cells.

There are several types of regulatory elements, including those that are developmentally regulated, inducible and constitutive. A regulatory element that is developmentally regulated, or controls the differential expression of a gene under its control, is activated within certain organs or tissues of an organ at specific times during the development of that organ or tissue. However, some regulatory elements that are developmentally regulated may preferentially be active within certain organs or tissues at specific developmental stages, they may also be active in a developmentally regulated manner, or at a basal level in other organs or tissues within a plant as well.

By "promoter" it is meant the nucleotide sequences at the 5' end of a coding region, or fragment thereof that contain all the signals essential for the initiation of transcription and for the regulation of the rate of transcription. There are generally two types of promoters, inducible and constitutive promoters. If tissue specific expression of the gene is desired, for example seed, or leaf specific expression, then promoters specific to these tissues may also be employed.

An inducible promoter is a promoter that is capable of directly or indirectly activating transcription of one or more DNA sequences or genes in response to an inducer. In the absence of an inducer the DNA sequences or genes will not be transcribed. Typically the protein factor that binds specifically to an inducible promoter to activate transcription is present in an inactive form which is then directly or indirectly converted to the active form by the inducer. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible promoter may be exposed to an inducer by externally applying the inducer to the cell or plant such as by spraying, watering, heating or similar methods. Examples of inducible promoters include plant promoters such as: the alfalfa plastocyanine promoter (see, for example WO01/025455), which is light-regulated; the alfalfa nitrite reductase promoter (NiR; see for example WO01/025454, which can be induced by fertilization with nitrates (3); and the alfalfa dehydrine promoter (US Application Serial Number 60/757,486), which is induced by environmental stresses such as cold.

A constitutive promoter directs the expression of a gene throughout the various parts of a plant and continuously throughout plant development. Any suitable constitutive promoter may be used to drive the expression of rHA within a transformed cell, or all organs or tissues, or both, of a host organism. Examples of known constitutive promoters include those associated with the CaMV 35S transcript. (Odell et al., 1985, Nature, 313: 810-812), the rice actin 1 (Zhang et al, 1991, Plant Cell, 3: 1155-1165) and triosephosphate isomerase 1 (Xu et al, 1994, Plant Physiol. 106: 459-467) genes, the maize ubiquitin 1 gene (Cornejo et al, 1993, Plant Mol. Biol. 29: 637-646), the *Arabidopsis* ubiquitin 1 and 6 genes (Holtorf et al, 1995, Plant Mol. Biol. 29: 637-646), and the tobacco translational initiation factor 4A gene (Mandel et al, 1995 Plant Mol. Biol. 29: 995-1004).

The term "constitutive" as used herein does not necessarily indicate that a gene is expressed at the same level in all cell types, but that the gene is expressed in a wide range of cell types, although some variation in abundance is often observed.

The chimeric gene construct of the present invention can further comprise a 3' untranslated region. A 3' untranslated region refers to that portion of a gene comprising a DNA segment that contains a polyadenylation signal and any other regulatory signals capable of effecting mRNA processing or gene expression. The polyadenylation signal is usually characterized by effecting the addition of polyadenylic acid tracks to the 3Y end of the mRNA precursor. Polyadenylation signals are commonly recognized by the presence of homology to the canonical form 5' AATAAA-3' although variations are not uncommon.

Examples of suitable 3' regions are the 3' transcribed non-translated regions containing a polyadenylation signal of *Agrobacterium* tumor inducing (Ti) plasmid genes, such as the nopaline synthase (*Nos* gene) and plant genes such as the soybean storage protein genes and the small subunit of the ribulose-1, 5-bisphosphate carboxylase (ssRUBISCO) gene. The 3' untranslated region from the structural gene of the present construct can therefore be used to construct chimeric genes for expression in plants. Other examples of suitable 3' regions are terminators, which may include, but are not limited to the noncoding 3' region of the sequence of the *plastocyanine* or the *nitrite reductase* or the *dehydrine* alfalfa genes.

The chimeric gene construct of the present invention can also include further enhancers, either translation or transcription enhancers, as may be required. These enhancer regions are well known to persons skilled in the art, and can include the ATG initiation codon and adjacent sequences. The initiation codon must be in phase with the reading frame of the coding sequence to ensure translation of the entire sequence. The translation control signals and initiation codons can be from a variety of origins, both natural and synthetic. Translational initiation regions may be provided from the source of the transcriptional initiation region, or from the structural gene. The sequence can also be derived from the regulatory element selected to express the gene, and can be specifically modified so as to increase translation of the mRNA.

Also considered part of this invention are plants, portion or tissues of plants, plant cells, trees, portion of trees, tree cells, yeast, bacteria, fungi, insect and animal cells containing the chimeric gene construct comprising a nucleic acid encoding rHA, in accordance with the present invention. However, it is to be understood that the regulatory elements of the present invention may also be combined with coding region of interest for expression within a range of host organisms that are amenable to transformation. Such host organisms include:
- plants, both monocots and dicots, for example, corn, cereal plants, wheat, barley, oat, tobacco, *Brassica,* soybean, bean, pea, alfalfa, potato, tomato, ginseng, *Arabidopsis;* a portion or tissue of these plants for example, leaves, roots, stems, meristem, floral structure, cells of these plants and
- yeast, fungi, insects, animal and bacteria cells.

Methods for the transformation and regeneration of these organisms are established in the art and known to one of skill in the art and the method of obtaining transformed and regenerated plants is not critical to this invention.

By "transformation" it is meant the interspecific transfer of genetic information (nucleotide sequence) that is manifested genotypically, phenotypically, or both. The interspecific transfer of genetic information from a chimeric construct to a host may be heritable and the transfer of genetic information considered stable, or the transfer may be transient and the transfer of genetic information is not inheritable.

Methods of regenerating whole plants from plant cells are also known in the art. In general, transformed plant cells are cultured in an appropriate medium, which may contain selective agents such as antibiotics, where selectable markers are used to facilitate identification of transformed plant cells. Once callus forms, shoot formation can be encouraged by employing the appropriate plant hormones in accordance with known methods and the shoots transferred to rooting medium for regeneration of plants. The plants may then be used to establish repetitive generations, either from seeds or using vegetative propagation techniques.

The constructs of the present invention can be introduced into plant cells using Ti plasmids, Ri plasmids, plant virus vectors, direct DNA transformation, microinjection, electroporation, etc. For reviews of such techniques see for example Weissbach and Weissbach, Methods for Plant Molecular Biology, Academy Press, New York VIII, pp. 421-463 (1988); Geierson and Corey, Plant Molecular Biology, 2d Ed. (1988); and Miki and Iyer, Fundamentals of Gene Transfer in Plants. In Plant Metabolism, 2d Ed. DT. Dennis, DH Turpin, DD Lefebrve, DB Layzell (eds), Addison Wesly, Langmans Ltd. London, pp. 561-579 (1997). Other methods include direct DNA uptake, the use of liposomes, electroporation, for example using protoplasts, microinjection, microprojectiles or whiskers, and vacuum infiltration. See, for example, Bilang, et al. (Gene 100: 247-250 (1991), Scheid et al. (Mol. Gen. Genet. 228: 104-112, 1991), Guerche et al. (Plant Science 52: 111-116, 1987), Neuhause et al. (Theor. Appl Genet. 75: 30-36, 1987), Klein et al., Nature 327: 70-73 (1987); Howell et al. (Science 208: 1265, 1980), Horsch et al. (Science 227: 1229-1231, 1985), DeBlock et al., Plant Physiology 91: 694-701, 1989), Methods for Plant Molecular Biology (Weissbach and Weissbach, eds., Academic Press Inc., 1988), Methods in Plant Molecular Biology (Schuler and Zielinski, eds., Academic Press Inc., 1989), Liu and Lomonossoff (J Virol Meth, 105:343-348, 2002,), U.S. Pat. Nos. 4,945,050; 5,036,006; and 5,100,792, U.S. patent application Ser. Nos. 08/438,666, filed May 10, 1995, and 07/951,715, filed Sep. 25, 1992.

As described below, transient expression methods may be used to express the constructs of the present invention (see Liu and Lomonossoff, 2002, Journal of Virological Methods, 105:343-348. Alternatively, a vacuum-based transient expression method, as described by Kapila et al., 1997 may be used. These methods may include, for example a method of Agro-inoculation or Agro-infiltration, syringe infiltration, however, other transient methods may also be used as noted above. With Agro-inoculation, Agro-infiltration, or synringe infiltration, a mixture of *Agrobacteria* comprising the desired nucleic acid enter the intercellular spaces of a tissue, for example the leaves, aerial portion of the plant (including stem, leaves and flower), other portion of the plant (stem, root, flower), or the whole plant. After crossing the epidermis the *Agrobacteria* infect and transfer t-DNA copies into the cells. The t-DNA is episomally transcribed and the mRNA translated, leading to the production of the protein of interest in infected cells, however, the passage of t-DNA inside the nucleus is transient.

To aid in identification of transformed plant cells, the constructs of the present invention may be further manipulated to include plant selectable markers. Useful selectable markers include enzymes which provide for resistance to an antibiotic such as gentamycin, hygromycin, kanamycin, and the like. Similarly, enzymes providing for production of a compound identifiable by colour change such as *GUS* (β-glucuronidase), or luminescence, such as luciferase are useful.

When specific sequences are referred to in the present invention, it is understood that these sequences include within their scope sequences that are "substantially homologous" to the specific sequences, or sequences or a compliment of the sequences hybridise to one or more than one nucleotide sequence as defined herein under stringent hybridisation conditions. Sequences are "substantially homologous" when at least about 70%, or more preferably 75% of the nucleotides match over a defined length of the nucleotide sequence providing that such homologous sequences exhibit one or more than one regulatory element activity as disclosed herein.

Such a sequence similarity may be determined using a nucleotide sequence comparison program, such as that provided within DNASIS (using, for example the following parameters: GAP penalty 5, #of top diagonals 5, fixed GAP penalty 10, k-tuple 2, floating gap 10, and window size 5). However, other methods of alignment of sequences for comparison are well-known in the art for example the algorithms of Smith & Waterman (1981, Adv. Appl. Math. 2:482), Needleman & Wunsch (J. Mol. Biol. 48:443, 1970), Pearson & Lipman (1988, Proc. Nat'l. Acad. Sci. USA 85:2444), and by computerized implementations of these algorithms (GAP, BESTFIT, FASTA, and BLAST, available through the NIH.), or by manual alignment and visual inspection (see, e.g., Current Protocols in Molecular Biology, Ausubel et al., eds. 1995 supplement), or using Southern or Northern hybridization under stringent conditions (see Maniatis et al., in Molecular Cloning (A Laboratory Manual), Cold Spring Harbor Laboratory, 1982). Preferably, sequences that are substantially homologous exhibit at least about 80% and most preferably at least about 90% sequence similarity over a defined length of the molecule.

An example of one such stringent hybridization conditions may be overnight (from about 16-20 hours) hybridization in 4 X SSC at 65°C, followed by washing in 0.1 X SSC at 65°C for an hour, or 2 washes in 0.1 X SSC at 65°C each for 20 or 30 minutes. Alternatively an exemplary stringent hybridization condition could be overnight (16-20 hours) in 50% formamide, 4 X SSC at 42°C, followed by washing in 0.1 X SSC at 65°C for an hour, or 2 washes in 0.1 X SSC at 65°C each for 20 or 30 minutes, or overnight (16-20 hours), or hybridization in Church aqueous phosphate buffer (7% SDS; 0.5M NaPO₄ buffer pH 7.2; 10 mM EDTA) at 65°C, with 2 washes either at 50°C in 0.1 X SSC, 0.1% SDS for 20 or 30 minutes each, or 2 washes at 65°C in 2 X SSC, 0.1% SDS for 20 or 30 minutes each for unique sequence regions.

The rHA of the present invention can be used in conjunction with existing influenza vaccines, to supplement the vaccines, render them more efficacious, and to reduce the administration dosages necessary. As would be known to a person of skill in the art, the vaccine may be directed against one or more than one influenza virus. Examples of suitable vaccines include those commercially available from Sanofi-Pasteur, ID Biomedical, Merial, Sinovac, Chiron, Roche, MedImmune, GlaxoSmithKline, and the like.

### Example 1: rHA strategy

The HA chosen to exemplify the present invention was obtained from influenza strain A/New Caledonia/20/99 (H1N1). The A/New Caledonia/20/99 HA has been well characterized and immunodetection tools are commercially available. As the three-dimensional structures of all HAs are well conserved, the present expression strategy can directly apply to any other HA.

The expression of rHA was directed to the ER and the secretory pathway using the signal peptide of the alfalfa PDI (SEQ ID NO:3). This signal peptide was fused directly to the N-terminal primary sequence of mature rHA (Figure 4; SEQ ID NO:8). The theoretical cleavage of the signal peptide from mature rHA with the PDI signal peptide was verified using the SignalP server 3.0.

rHA can be retained in the ER using the SEKDEL (SEQ ID NO:4) or the HDEL (SEQ ID NO:5) retention signals, both fused to the C-terminal end of rHA protein sequence.

When the GCN4-pII peptide was used as the oligomerization domain, it was fused directly to C-terminal of the last Tyr residue of HA₀. The Met residue at position 1 of GCN4-pII was changed to a Leu because Leu is more inert than Met and it has similar packing volume compared to the original Met. Amino acids Ser-Ala-Ala were added at the C-terminus of GCN4-pII.

When the PRD of maize gamma-γ-Zein was used, a peptide with 8 repetitions of the PPPVHL (SEQ ID NO:2) was fused to HA, to replace the transmembrane domain of HA. The 4 first proline residues were also included in the fusion. The C-terminal cysteine was excluded. To accommodate the fusion of a polyproline helix to the C-terminus of HA, the peptide Gly-Gly-Ala-Gly (SEQ ID NO:3) was added the N-terminal of PRD. The Ser-Ala-Ala peptide was added to the C-terminus of PRD.

In total, 8 different rHA gene constructs were prepared to test their expression *in planta:*
1. The full length rHA, including the transmembrane domain, with the PDI signal peptide (SEQ ID NO:9);
2. The transmembrane domain and cytoplasmic tail were removed from the rHA of item 1 and replaced by the retention signal SEKDEL (SEQ ID NO:10);
3. The transmembrane domain and cytoplasmic tail were removed from the rHA of item 1 and replaced by the retention signal HDEL (SEQ ID NO:11);
4. The transmembrane domain and cytoplasmic tail were removed from the rHA of item 1 (SEQ ID NO:12);
5. The transmembrane domain and cytoplasmic tails were removed from the rHA of item 1 and replaced by the GCN4-pII (SEQ ID NO:13);
6. The transmembrane domain and cytoplasmic tails were removed from the rHA of item 1 and replaced by the GCN4-pII, with the SEKDEL retention signal at the C-terminus (SEQ ID NO:14);
7. The transmembrane domain and cytoplasmic tails were removed from the rHA of item 1 and replaced by the PRD domain (SEQ ID NO:15); and
8. The transmembrane domain and cytoplasmic tails were removed from the rHA of item 1 and replaced by the PRD domain, with the SEKDEL retention signal at the C-terminus (SEQ ID NO:16).

### Example 2: Gene synthesis

The eight different gene constructs described in Example 1 were synthesized. The wild-type nucleotide sequence of HA from influenza strain A/New Caledonia/20/99 was only modified to insert or remove restriction sites to facilitate the cloning steps. These minor modifications in the gene sequence did not change the resulting amino acid sequence of the protein. The HA₀ gene was synthesized with an ApaI restriction site at the 5'-end, in phase with the ATG of the PDI signal peptide. The 7 other genes bear a KpnI restriction site at their 5'-end. Every gene ends at the 3'end with a stop codon (TAA), followed by a SacI and a StuI restriction sites. According to the designed cloning strategy, the following restriction sites were design to be unique in the final plasmid: ApaI, BstZ17 I, Sac I, Kpn I, Bgl II, and Stu I. The different gene fragments were synthesized at the Plant Biotechnology Institute and were delivered cloned into pUC18. Selected nucleotide sequence information is given in Figure 6 (SEQ ID NOs: 17-24).

### Example 3: Genetic assembly of vectors expressing rHA fusions

The various DNA constructs were assembled using known recombinant DNA techniques (Sambrook et al (1989) Molecular cloning: A laboratory Manual, Sec Ed. Cold Spring Harbor Laboratory press, Cold Spring Harbor, NY). The E. coli DH5α strain was used has a host for amplification of the binary vectors, unless otherwise specified. The HA₀ gene fragment was ligated directly into the plastocynine cassette (already containing the pdi signal peptide) using the bglII and SacI restriction sites, or was co-ligated with the 7 other C-terminus ends using the KpnI, bglII and SacI restriction sites. In each case, the gene was inserted into a plastocyanine expression cassette contained on transfer DNA (t-DNA) of a binary vectors. t-DNA is the part of the binary vector that is integrated in the genome (from the right to the left border) of the host plant cell upon transformation. The binary vectors used in this example were derived from the commercial plasmid pCAMBIA 2300 (Cambia, Canberra, Au) and contained, from the right to left borders sequences of the t-DNA, the following elements:
- various proprietary expression cassettes (promoter and terminator), available from Medicago, where the rHA genes are inserted;
- a multiple cloning site to facilitate the cloning; and
- the gene coding for neomycin phosphotansferase II (nptII), a selectable marker that confers resistance to kanamycin, under the control of a nopaline synthase (NOS) promoter and a cauliflower 35S mosaic virus (35S) terminator.

Each clone was submitted to DNA sequencing to verify the nucleotide sequence of the clones. Nucleotide variations were tolerated in the sequence of the rHA gene, provided the resulting amino acid sequence still encoded the exact same protein sequence (SEQ ID NOs:9-16). The 8 different DNA constructs are listed in Table 1, and an example of the resulting rHA t-DNA is shown schematically in Figure 7.

**Table 1: DNA constructs for rHA**

| No. | Expression strategy | Sub-cellular targeting | ER retention signal | SEQ ID NO: |
|---|---|---|---|---|
| 540 | Complete transmembrane secreted | Apoplast | | 9 |
| 541 | ER-retained | ER | SEKDEL | 10 |
| 542 | ER-retained | ER | HDEL | 11 |
| 543 | Soluble, secreted | Apoplast | | 12 |
| 544 | Soluble trimeric GCN4 | Apoplast | | 13 |
| 545 | Soluble trimeric GCN4 | ER | SEKDEL | 14 |
| 546 | Soluble trimeric PRD | Apoplast | | 15 |
| 547 | Soluble trimeric PRD | ER | SEKDEL | 16 |

DNA isolated from *E. coli* proper clones was finally inserted into *Agrobacterium tumefaciens* Agl1 or other strains. The list of the *Agrobacterium* clones containing the various t-DNAs used in the present invention appears at Table 1. In preparation for plant transient expression experiments, plasmidic DNA was isolated from *Agrobacterium* clones and submitted restriction digest mapping to ensure integrity of the DNA

### Example 3: Transient expression of rHA in tobacco

***Agrobacterium growth***. The *Agrobacterium* clones containing a binary vector bearing rHA DNA constructs (Table 1) were grown for 24 hours at 28°C in 2 mL of YEB or LB medium containing 25 and 50 µg/mL of each carbenicilin and kanamycin, respectively. 10 µL of these cultures were used as starting inoculums to generate cultures of 25 mL of YEB induction medium (YEB medium, 10 mM 2 (N morpholino) ethanesulfonic acid (MES), pH adjusted to 5.6, 25 mg/L carbenicillin, 50 mg/L kanamycin, 20 µM acetosyringone). The latter were grown in rotary shaker (220 rpm) incubator at 28°C for 18 hours or until an optical density at 600 nm (OD600) of 0.8 to 1 was reached.

***Growth of non-transgenic tobacco.** Nicotiana benthamiana* and *Nicotiana tabacum* plants were grown from seeds in a peat-based substrate (AgroMix) in a greenhouse. Seedlings were initially raised in a nursery and later transplanted to pots. Plants were irrigated twice a day and receive 180 ppm of nitrogen at each application. Greenhouse condition were kept at 25°C during the day and 21°C during the night, under a long day photoperiod regime (16 h light/8 h dark cycles), with artificial lightning of 20 watt m-2 at plant level.

***Transient expression of rHA tobacco.*** The *Agrobacterium* culture prepared as described earlier was centrifuged 8 min at 10 000 g, resuspended in the same volume of inoculation medium (10 mM MgCl2, 10 mM MES, adjusted to pH 5.6, and supplemented with 100 µM acetosyringone) and kept at room temperature (RT, 23°C) for 1h prior to inoculation. Alternatively, the suspension can be kept at 4°C for 24 hours prior to inoculation. Transient transformation of *N. benthamiana* and *N. tabacum* were essentially performed as described in Liu and Lomonossoff (2002, Journal of Virological Methods, 105:343-348), with the following modifications. For the expression of rHA, a mixture of two *Agrobacteria* strains was inoculated. The first strain contained one of the clones described at Table 1 and the second strain contained the HcPro suppressor of silencing from the Potato Virus Y under the control of the 35S promoter. After inoculation, the plants were incubated in a greenhouse. Temperature was kept at a minimum 23°C during the day and 21°C during the night. Plants were irrigated twice a day and received 180 ppm of nitrogen at each application. Harvest of biomass was undertaken after 4-8 days.

### Example 5: Demonstration of expression of rHA in tobacco leaves

***Preparation of a soluble protein extracts from inoculated leaves.*** Leaves were analyzed directly after harvesting or after freezing the biomass at -80°C. A vegetable biomass of Agro-inoculated leaves of ∼0.1 g was weighted and used for analysis of rHA transient expression.

One of two extraction methods was used to generate a total protein extract: by grounding the vegetable tissue with a mortar and a pestle, or by pulverizing it in a MixerMill300 (MM300) from Retsch. 0.1 g of plant biomass was transferred into a clean and pre-cooled mortar. 0.3 mL of cold extraction buffer (Tris 50 mM pH 7.4 containing NaCl 150 mM, 0.1% Triton X-100, and 5% glycerol) was added as well as PMSF and chymostatin to final concentrations of 1 mM and 10 µM, respectively. Leaves were ground with a pestle until a homogeneous preparation was obtained. The plant extract was then transferred into a 1.5 mL microtube and centrifuged at 20,000g for 20 min at 4°C. Alternatively, 0.1g of plant tissue with 0.3 mL of extraction buffer was introduced into non-sterile 1.5 microtube. A tungsten bead was added to each tube. The box was submitted to 3 min cycle of agitation at 30 Hz. The cycle was repeated 2 times. The plant extracts were then centrifuged as described above.

Following centrifugation, the supernatant was transferred into a clean microtube and maintained on ice. Finally, the total protein content of individual protein extracts was measured by the Bradford method using BSA as the reference protein.

***Immunodetection of rHA transiently expressed in tobacco leaf material***. The Agro-inoculated tobacco leaf protein extracts was prepared as described above. The following 3 controls were also prepared: 1) 1 ng of pure rHA (recombinant rHA produced by CHO cells, Protein Sciences Corporation, catalog # 3006) to which 1 µg of BSA (Pierce) was added, 2) 2-10 µg of tobacco protein extract obtained from a biomass agro-inoculated with agrobacteria transformed with an empty pCAMBIA plasmid, and 3) 1 ng of pure rHA spiked to 2-10 µg of the protein extract of sample #2.

Typically, 2-10 µg of protein extract from each biomass (transformed with one of the 8 different DNA constructs, see Table 1) was loaded on SDS-PAGE for Western blot analysis. Samples were boiled for 5 min at 100°C prior to loading on denaturing SDS-PAGE (concentration 6% acrylamide; separation 10% acrylamide, Tris-glycine buffer) as essentially described by Thomas (1975, Proc. Nat. Acad. Sci. USA, 72:2626-2630). Electrophoresis was performed at 110 volts for 90 min using Mini Protean III apparatus (BioRad). Separated proteins were then blotted onto a PVDF membrane in a Tris 25 mM, Glyine 192 mM, Methanol 10 % (v/v) pH 8.3 buffer at 100 V for 30 min or 60 min when using a Criterion blotter or a Mini-trans blot Unit (BioRad), respectively.

Immunodetection of rHA was achieved with the following antibodies. A mouse monoclonal antibody to influenza A virus (Fitzgerald Industries International Inc. Catalog # 10- I50) was used as the primary antibody at 1/1,000. The secondary antibody was a peroxidase conjugated goat anti-mouse IgG (H + L) (Jackson ImmunoResearch #111-035-146) used at 1/10,000. The peroxydase activity was detected using Immobilon Western Chemiluminescent HRP substrate (Milipore, catalog # WBRL50100) or BM chemiluminescent blotting substrate (POD) (Roche, catalog #11500 694 001).

The immunodetection method was optimized to obtain a specific signal against each rHA, without cross-reactivity with endogenous plant proteins. The limit of detection was low; which allowed detection of the proteins expressed at levels as low as >0.004 % (1 ng into 25 µg of extract) of total soluble proteins (TSP).

As shown in Figure 8, rHA was expressed in every condition tested and in both tobacco species. The use of non-reducing conditions allowed detection of different oligomeric forms of hemagglutinin: monomers, dimers and trimers. With decreasing quantities of protein loaded on the gel, as examplified at Figure 9, the oligormeric forms of rHA are resolved. The latter are the same for every construct tested and in both tobacco species: immunodetection signals are observed at the level of the trimer and of the monomer. A third immunoreactive band is also observed, which migrates between the expected molecular weight of the dimer and that of the trimer.

The replacement of the transmembrane domain by the different trimeric coil-coiled peptides allowed expression of rHA with expression levels equal to or higher than the transmembrane form of rHA. This is especially true for the fusion with GCN4-pII. The fusion of rHA with the PRD allows expression at lower levels compared to the GCN4-pII fusion. The expression levels of the GCN4-pII-rHA fusion is of, at least, 0.01% TSP (>1ng of rHA in 10 µg of TSP). Accumulation of rHA fusions appears higher in *N*. *benthamiana* compared to *N*. *tabacum.* Finally, retention of rHA to the ER, for every construct tested, increases significantly the expression levels of rHA. This tendency has been observed for many other proteins. The ER subcellular space is generally recognized for its low proteolytic activity compared to other compartments such as the apoplast, resulting in higher accumulation of recombinant proteins.

The same protein extracts were analysed under reducing conditions and immunodetected as described earlier to investigate whether the HA₀ chain is proteolysed into the two-chain form of rHA, i.e., HA1 and HA2. The expected molecular weights of the latter are 47 and 28 kDa, respectively. It is shown at Figure 10 that the HA₀ is not readily converted to a two-chain form. It is worth noticing that proteolysis or clipping of rHA appears to be greater in the *N*. *tabacum* extracts.

### Example 6: Demonstration of trimeric assembly of rHA

***Hemagglutination test***. The hemagglutination assay was performed essentially as described by Nayak et al. (2004, J. Virol Methods, 122:9-15), with the following modifications. Extracts were prepared by homogenization of tissue in 50 mM Tris pH 7.4, 150 mM NaCl, 0.1% Triton X-100, and containing PMSV 1 MM and Chymostatin 10 µM as protease inhibitors. Soluble fractions were diluted to 0.2 mg/mL in PBS. Serial double dilutions of the tamples (100 µL) were made in round-bottomed 96-well microplate containing 100 µL of PBS. 100 µL of red blood chicken cells (2X10⁷ rbc/mL) (Innovative Research, South Field, MI, USA) were added to each well. The plate was then incudated for 60-120 min at room temperature. Assay includes the following positive controls: rHA from PSC, ranged from 1 µg to 7.8 pg, diluted in PBS or spiked in non-transformed extract. A negative control using non-transformed extract was also run.

The hemagglutination assay was used to demonstrate the trimeric assembly of rHA fused to the coiled-coli peptides: That test, although very simple, is used on a routine basis to quantify the influenza titer. The principle of the assay is: free red blood cell roll will roll at the bottom an ELISA well, forming a cellular pile of red blood cells that can be easily detected. If hemagglutination occurs, the blood cell will form a matrix so the the blood cell can not roll at the bottom the ELISA well and no point can be seen. Figure 11 shows that hemagglutination of chicken red blood cell can be performed directly on rHA expressed in a plant extract. More over the hemagglutination can occur with a plant extract expressing the rHA fused to GCN4-pII. Since the rHA monomer is unable to undergo red blood cell hemagglutination, that result ultimately demonstrates that the chimeric rHA assemble itself as a trimer in the plant.

Table 2 summarizes the sequences listed in the present application.

**Table 2: Sequence description for sequence identifiers.**

| **SEQ ID NO:** | **Sequence description** |
|---|---|
| 1 | Amino acid sequence of the GCN4-pII peptide |
| 2 | peptide repeat of the PRD peptide of maize gamma-γ-Zein |
| 3 | tetrapeptide linker |
| 4 | SEKDEL ER retention signal |
| 5 | HDEL ER retention signal |
| 6 | amino acid sequence of the alfalfa PDI signal peptide |
| 7 | nucleotide sequence of the alfalfa PDI signal peptide |
| 8 | Amino acid sequence of mature HA |
| 9 | full length rHA, including the transmembrane domain, with the HA signal peptide replaced by the PDI signal peptide |
| 10 | SEQ ID NO:9, with the transmembrane domain and cytoplasmic tail replaced by the retention signal SEKDEL |
| 11 | SEQ ID NO:9, with the transmembrane domain and cytoplasmic tail replaced by the retention signal HDEL; |
| 12 | SEQ ID NO:9, without the transmembrane domain and cytoplasmic tail |
| 13 | SEQ ID NO:9, with the transmembrane domain and cytoplasmic tail replaced by the GCN4-pII peptide |
| 14 | SEQ ID NO:13, with the SEKDEL retention signal at the C-terminus |
| 15 | SEQ ID NO:9, with the transmembrane domain and cytoplasmic tail replaced by the PRD domain |
| 16 | SEQ ID NO:15, with the SEKDEL retention signal at the C-terminus |
| 17 | the nucleotide sequence of the HA₀ gene fragment |
| 18 | the nucleotide sequence of the transmembrane domain and the cytoplasmic tail gene fragment |
| 19 | the nucleotide sequence of the ER-retained SEKDEL gene fragment. |
| 20 | the nucleotide sequence of the ER-retained HDEL gene fragment |
| 21 | the nucleotide sequence of the GCN4-pII gene fragment |
| 22 | the nucleotide sequence of the ER-retained GCN4-pII gene fragment |
| 23 | the nucleotide sequence of the PRD gene fragment |
| 24 | the nucleotide sequence of the ER-retained PRD gene fragment |

## Claims

1. A recombinant hemagglutinin (rHA), comprising:
a) a hemagglutinin domain selected from the group consisting of hemagglutinin subtypes H1, H2, H3, H4, H5, H6, H7, H8, H9, H10, H11, H12, H13, H14, H15, and H16; and
b) an oligomerization domain selected from the group consisting of a GCN4-pII peptide and a proline rich domain (PRD) of maize γ-zein, wherein amino acid residues are added to the C-terminal end of the oligomerization domain such that the oligomerization domain does not terminate in an α-helix;
c) signal peptide
d) an endoplasmic reticulum (ER) retention signal and
wherein the rHA is produced as a chimeric soluble homotrimer.

2. The rHA of claim 1, wherein the added amino acid residues are Ser-Ala-Ala.

3. The rHA of claim 1 or 2, wherein the ER retention signal is SEKDEL or HDEL.

4. The rHA of any one of claims 1-3, wherein the PRD has 4, 6 or 8 peptide repeats.

5. The rHA of claim 1, wherein the signal peptide is a protein disulfide isomerase (PDI) signal peptide.

6. A nucleotide sequence encoding the rHA of any one of claims 1 to 5.

7. A vector comprising the nucleic acid of claim 6.

8. A host cell expressing the rHA of any one of claims 1 to 5.

9. A host cell transformed with the nucleic acid of claim 6.

10. A host cell transformed with the vector of claim 7.

11. A method of producing rHA, comprising providing a host cell comprising the nucleic acid of claim 6, and expressing the rHA in the host cell.

12. A method of expressing rHA within a plant comprising introducing a vector of claim 7 into the plant and expressing the rHA.

13. The method of claim 12, wherein the nucleic acid is introduced in the plant in a transient manner or the nucleic acid is introduced in the plant so that it is stable.

14. A method of producing a recombinant hemagglutinin (rHA) in a plant comprising:
a) introducing a nucleic acid sequence into the plant, or portion thereof, the nucleic acid sequence comprising a regulatory region operatively linked to the nucleic acid of claim 6 and
b) growing the transgenic plant, thereby producing the rHA.

15. The method of claim 14, wherein in the step of introducing (step a), the nucleic acid is introduced in the plant in a transient manner or the nucleic acid is introduced in the plant so that it is stable.

## Patentansprüche

1. Rekombinantes Hämagglutinin (rHA), umfassend:
a) eine Hämagglutinin - Domäne ausgewählt aus der Gruppe bestehend aus Hämagglutininsubtypen H1, H2, H3, H4, H5, H6, H7, H8, H9, H10, H11, H12, H13, H14, H15 und H16; und
b) eine Oligomerisierungsdomäne ausgewählt aus der Gruppe bestehend aus einem GCN4-pII-Peptid und einer Prolin - reichen Domäne (PRD) von Mais - γ - Zein, wobei Aminosäurereste an das C - terminale Ende der Oligomerisierungsdomäne hinzugefügt sind, so dass die Oligomerisierungsdomäne nicht in einer α - Helix endet;
c) Signalpeptid
d) ein endoplasmatisches Retikulum (ER) Retentionssignal und
wobei das rHA als chimäres lösliches Homotrimer hergestellt wird.

2. rHA gemäß Anspruch 1, wobei die hinzugefügten Aminosäurereste Ser-Ala-Ala sind.

3. rHA gemäß Anspruch 1 oder 2, wobei das ER Retentionssignal SEKDEL oder HDEL ist.

4. rHA gemäß einem der Ansprüche 1 - 3, wobei die PRD 4, 6 oder 8 Peptidwiederholungen aufweist.

5. rHA gemäß Anspruch 1, wobei das Signalpeptid ein Proteindisulfidisomerase (PDI) Signalpeptid ist.

6. Nukleotidsequenz, die das rHA gemäß einem der Ansprüche 1 bis 5 kodiert.

7. Vektor, der die Nukleinsäuren gemäß Anspruch 6 umfasst.

8. Gastzelle, die das rHA gemäß jedem der Ansprüche 1 bis 5 exprimiert.

9. Gastzelle, die mit der Nukleinsäure gemäß Anspruch 6 transformiert ist.

10. Gastzelle, die mit dem Vektor gemäß Anspruch 7 transformiert ist.

11. Verfahren zum Herstellen von rHA, umfassend Bereitstellen einer Gastzelle, die die Nukleinsäure gemäß Anspruch 6 umfasst, und Exprimieren des rHA in der Gastzelle.

12. Verfahren zum Exprimieren von rHA in einer Pflanze, das das Einführen eines Vektors gemäß Anspruch 7 in die Pflanze und Exprimieren des rHA umfasst.

13. Verfahren gemäß Anspruch 12, wobei die Nukleinsäure in transienter Weise in die Pflanze eingeführt wird oder die Nukleinsäure so in die Pflanze eingeführt wird, dass sie stabil ist.

14. Verfahren zum Herstellen eines rekombinanten Hämagglutinins (rHA) in einer Pflanze, umfassend:
a) Einführen einer Nukleinsäuresequenz in die Pflanze oder Teil davon, wobei die Nukleinsäuresequenz eine Regulationsregion, die wirkend mit der Nukleinsäure gemäß Anspruch 6 verbunden ist, umfasst, und
b) Wachsen lassen der transgenen Pflanze, dabei das rHA herstellend.

15. Verfahren gemäß Anspruch 14, wobei in dem Schritt des Einführens (Schritt a), die Nukleinsäure in transienter Weise eingeführt wird oder die Nukleinsäure in die Pflanze eingeführt wird, so dass sie stabil ist.

## Revendications

1. Hémagglutinine recombinante (rHA), comprenant :
a) un domaine d'hémagglutinine sélectionné dans le groupe consistant en les sous-types d'hémagglutinine H1, H2, H3, H4, H5, H6, H7, H8, H9, H10, H11, H12, H13, H14, H15 et H16 ; et
b) un domaine d'oligomérisation sélectionné dans le groupe consistant en un peptide GCN4-pII et un domaine riche en proline (PRD) de la γ-zéine du maïs, dans lequel des résidus d'acides aminés sont ajoutés à l'extrémité C-terminale du domaine d'oligomérisation de sorte que le domaine d'oligomérisation ne se termine pas par une hélice α;
c) un peptide signal
d) un signal de rétention dans le réticulum endoplasmique (RE) et
où la rHA est produite sous forme d'homotrimère soluble chimérique.

2. rHA selon la revendication 1, dans laquelle les résidus d'acides aminés ajoutés sont Ser-Ala-Ala.

3. rHA selon la revendication 1 ou 2, dans laquelle le signal de rétention dans le RE est SEKDEL ou HDEL.

4. rHA selon l'une quelconque des revendications 1 à 3, dans laquelle le PRD possède 4, 6 ou 8 répétitions peptidiques.

5. rHA selon la revendication 1, dans laquelle le peptide signal est un peptide signal de protéine disulfure isomérase (PDI).

6. Séquence de nucléotides codant pour la rHA selon l'une quelconque des revendications 1 à 5.

7. Vecteur comprenant l'acide nucléique selon la revendication 6.

8. Cellule hôte exprimant la rHA selon l'une quelconque des revendications 1 à 5.

9. Cellule hôte transformée avec l'acide nucléique selon la revendication 6.

10. Cellule hôte transformée avec le vecteur selon la revendication 7.

11. Procédé de production d'une rHA, comprenant la mise à disposition d'une cellule hôte comprenant l'acide nucléique selon la revendication 6, et l'expression de la rHA dans la cellule hôte.

12. Procédé d'expression d'une rHA au sein d'une plante, comprenant l'introduction d'un vecteur selon la revendication 7 dans la plante et l'expression de la rHA.

13. Procédé selon la revendication 12, dans lequel l'acide nucléique est introduit dans la plante de manière transitoire ou l'acide nucléique est introduit dans la plante de sorte qu'il soit stable.

14. Procédé de production d'une hémagglutinine recombinante (rHA) dans une plante, comprenant :
a) l'introduction d'une séquence d'acide nucléique dans la plante, ou une partie de celle-ci, la séquence d'acide nucléique comprenant une région régulatrice en liaison fonctionnelle avec l'acide nucléique selon la revendication 6 et
b) la mise en culture de la plante transgénique, en produisant ainsi la rHA.

15. Procédé selon la revendication 14, dans lequel à l'étape d'introduction (étape a), l'acide nucléique est introduit dans la plante de manière transitoire ou l'acide nucléique est introduit dans la plate de sorte qu'il soit stable.
